# EUROPEAN PATENT SPECIFICATION

(11) **EP 1 129 191 B1**
(45) Date of publication and mention of the grant of the patent: **22.03.2006**
(21) Application number: 99960975.3
(22) Date of filing: 12.11.1999
(51) Int. Cl.: C12N 15/12, C07K 14/47, C12Q 1/68, G01N 33/68, A61K 38/17

(54) **METHODS OF DIAGNOSING OR TREATING NEUROLOGICAL DISEASES**
VERFAHREN ZUR DIAGNOSE UND BEHANDLUNG NEUROLOGISCHER ERKRANKUNGEN
METHODES DE DIAGNOSTIC OU DE TRAITEMENT DE MALADIES NEUROLOGIQUES

(30) Priority: 12.11.1998 EP 98121478
(43) Date of publication of application: 05.09.2001
(73) Proprietor: EVOTEC Neurosciences GmbH, 22525 Hamburg (DE)
(72) Inventor: Nitsch, Roger, 8702 Zollikon (CH); Greeve, Isabell, 20143 Hamburg (DE)
(74) Representative: Meyers, Hans-Wilhelm
(86) International application number: PCT/EP1999/008744
(87) International publication number: WO 2000/029569

(56) References cited:
- EP-A- 0 814 157
- Emhum2 Database Entry Hsrsc390 Accession number D13643; 31 March 1993 NOMURA N.: "Human mRNA for KIAA0018 gene, complete cds." XP002099607 -& NOBUO NOMURA ET AL.: "Prediction of the coding sequences of unidentified human genes. I. The coding sequences of 40 new genes (KIAA0001-KIAA0040) deduced by analysis of randomly sampled cDNA clones from human immature myeloid cell line KG-1" DNA RESEARCH, vol. 1, no. 1, 1994, pages 27-35, XP002099608 -& NOBUO NOMURA ET AL.: "Prediction of the coding sequences of unidentified human genes. I. The coding sequences of 40 new genes (KIAA0001-KIAA0040) deduced by analysis of randomly sampled cDNA clones from human immature myeloid cell line KG-1 (Supplement)" DNA RESEARCH, vol. 1, no. 1, 1994, pages 47-56, XP002065816
- GREEVE, I. (1) ET AL: "Expression of Seladin -1, a novel neuroprotective gene with homologies to oxido-reductases is associated with selective vulnerability in Alzheimer's disease." SOCIETY FOR NEUROSCIENCE ABSTRACTS, (1999) VOL. 25, NO. 1-2, PP. 546. MEETING INFO.: 29TH ANNUAL MEETING OF THE SOCIETY FOR NEUROSCIENCE, PART MIAMI BEACH, FLORIDA, USA OCTOBER 23-28, 1999 THE SOCIETY FOR NEUROSCIENC. , XP002134188

## Description

Cell death is a common feature occuring in two distinct forms in nature. Necrosis results from physical or chemical insult while apoptosis or programmed cell death results from a self-destruction program within the cell in response to internal and external stimuli. Latter process is a gene-directed form of cell death that is essential for normal development and maintenance of multicellular organisms. Recent work has clearly demonstrated that dysregulation of apoptosis may underlie the pathogenesis of a variety of diseases. Apoptosis has been reported to occur in conditions characterized by ischaemia, e.g. myocardial infarction and stroke. It has been implicated in a number of liver disorders including obstructive jaundice. Hepatic damage due to toxins and drugs is also associated with apoptosis in hepatocytes. Apoptosis has also been identified as a key phenomenon in some diseases of the kidney, i.e. polycistic kidney, as well as in disorders of the pancreas like alcohol-induced pancreatitis and diabetes. AIDS and neurodegenerative disorders like Alzheimer's and Parkinson's disease represent the most widely studied group of disorders where an excess of apoptosis has been implicated. Amyotrophic lateral sclerosis, retinitis pigmentosa, epilepsy and alcoholic brain damage are other neurological disorders in which apoptosis has been implicated.

Neurological diseases are widely spread within a population and have a strong impact not only on patients' life but also on society as such. Therefore, there is a strong need to elucidate the causes and the underlying pathogenesis of such neurological diseases. Among such neurological diseases, Alzheimer's disease (AD) has a predominant position. Alzheimer's disease, first described by the Bavarian psychiatrist Alois Alzheimer in 1907, is a progressive neurological disorder which begins with short term memory loss and proceeds to loss of cognitive functions, disorientation, impairment of judgement and reasoning and, ultimately, dementia. It is the most common cause of dementia. AD has been estimated to afflict 5 to 11 percent of the population over age 65 and as much as 47 percent of the population over age 85. Moreover, as adults, born during the population boom of the 1940's and 1950's, approach the age when AD becomes more prevalent, the control and treatment of AD will become an even more significant health care problem. Familial forms of AD are genetically heterogeneous, but most with early onset are linked to mutations in the presenilin genes *PSEN1* and *PSEN2,* as well as to mutations of the amyloid precursor gene *APP.* The majority of AD patients have no obvious family history and are classified as sporadic AD. The neuropathology of AD is characterized by a substantial loss of neurons and synapses, and by the formation in brain of amyloid plaques and neurofibrillary tangles. Amyloid plaques are evenly distributed throughout the neocortex and the hippocampus, whereas neurodegeneration occurs predominantly in the inferior temporal lobes, the entorhinal cortex, and the hippocampus. Similar neurons in the frontal, parietal, and occipital lobes are largely preserved from degeneration even in severe end-stage AD. These observations indicate selective vulnerability of specific population of neurons. Factors that determine selective vulnerability of neurons in AD brains are unknown.

In a new approach for the systematic identification of unidentified expressed human genes, Nomura et al. (*DNA Research.* 1:27-35, 1994) constructed a medium-sized cDNA library from size-fractionated human cDNA. The authors used a human immature myeloid cell line (KG-1) as a source of mRNA for library construction. Clones containing unreported sequences at their 5'-termini and inserts in the range of 2-6 kb were isolated, and their nucleotide sequences were determined. Thus, nearly full-length transcripts of 40 new genes were identified and their possible coding regions predicted by computer analysis. According to Nomura et al., one of those reported genes, originally designated KIAA0018 (Emhum2 database entry Hsrsc390: accession number D13643), is transcribed into a cDNA of 4186 bp in length, with a potential protein coding region of 402 amino acids. Nomura et al. reported an ubiquitous expression of KIAA0018 mRNA in 16 different human tissues. The genomic location of the KIAA0018 gene was mapped to human chromosome 1

To elucidate the causes of cell degeneration and cell death is a general aim of the present invention. More specifically, the present invention aims at elucidating the causes and the underlying pathogenesis of neurological diseases, in particular Alzheimer's disease. It is therefore an object of the present invention to provide an insight into the pathogenesis of neurological diseases and to provide methods and materials which are suited for diagnosis and treatment of said diseases, cell degeneration and cell death.

The invention features an isolated nucleic acid molecule encoding a protein molecule whose amino acid sequence comprises the sequence shown in SEQ ID NO. 1 as well as the protein molecule according to SEQ ID NO.1. Hereinafter, the protein molecule of SEQ ID NO. 1 is denoted "SELADIN-1". One function of SELADIN-1 is to protect cells against degeneration and cell death. In particular, cells of the nerve system, muscular system, prostate, stomach, testis, ovary, adrenal glands, mammary glands, liver, spleen, lung, trachea or placenta are protected against degeneration and/or cell death. Therefore, the present invention also features functional variants of SELADIN-1 which might have a modification of the given primary structure of SELADIN-1, but whose essential biological function remains unaffected. "Variants" of a protein molecule shown in SEQ ID NO.1 include for example proteins with conservative amino acid substitutions .

in highly conservative regions. For example, isoleucine, valine and leucine can each be substituted for one another. Aspartate and glutamate can be substituted for each other. Glutamine and asparagine can be substituted for each other. Serine and threonine can be substituted for each other. Amino acid substitutions in less conservative regions include e.g.: Isoleucine, valine and leucine can each be substituted for one another. Aspartate and glutamate can be substituted for each other. Glutamine and asparagine can be subsituted for each other. Serine and threonine can be substituted for each other. Glycine and alanine can be substituted for each other. Alanine and valine can be substituted for each other. Methionine can be substituted for each of leucine, isoleucine or valine, and vice versa. Lysine and arginine can be substituted for each other. One of aspartate and glutamate can be substituted for one of arginine or lysine, and vice versa. Histidine can be substituted for arginine or lysine, and vice versa. Glutamine and glutamate can be substituted for each other. Asparagine and aspartate can be substituted for each other. Other examples of protein modifications include glycosilation and further posttranslational modifications. The invention also features the nucleic acid molecules encoding such functional variants of the protein molecule of SEQ ID NO. 1. Nucleic acid molecules can be DNA molecules, such as genomic DNA molecules or cDNA molecules, or RNA molecules, such as mRNA molecules. In particular, said nucleic acid molecule can be a cDNA molecule comprising a nucleotide sequence of SEQ ID NO. 2. The invention also features an isolated D N A molecule capable of hybridizing with the complement of the cD N A described in SEQ ID NO. 2 under stringent conditions. Examples for stringent conditions include (i) 0.2xSSC (standard saline citrate) and 0.1 % SDS at 60 °C and (ii) 50 % formamide, 4xSSC, 50 mM HEPES, pH 7.0, 10x Denhardt's solution, 100 µg/ml thermally denatured salmon sperm DNA at 42 °C.

In another aspect, the invention features a vector comprising a nucleic acid encoding a protein molecule shown in SEQ ID NO. 1. It also features a vector comprising a nucleic acid molecule encoding a protein molecule, the function of which is to protect cells against degeneration and/or cell death, wherein the amino acid sequence of the protein molecule comprises the sequence shown in SEQ ID NO. 1 or a functional variant thereof. In preferred embodiments, a virus, a bacteriophage, or a plasmid comprises the described nucleic acid. In particular, a plasmid adapted for expression in a bacterial cell comprises said nucleic acid molecule, e.g. a nucleic acid molecule encoding a protein molecule shown in SEQ ID NO. 1, and the regulatory elements necessary for expression of said molecule in the bacterial cell. In a further aspect, the invention features a plasmid adapted for expression in a yeast cell which comprises said nucleic acid molecule, e.g. a nucleic acid molecule encoding a protein molecule shown in SEQ ID NO. 1, and the regulatory elements necessary for expression of said molecule in the yeast cell. In another aspect, the invention features a plasmid adapted for expression in a mammalian cell which comprises a nucleic acid molecule, e.g. a nucleic acid molecule encoding a protein molecule shown in SEQ ID NO.1, and the regulatory elements necessary for expression of said molecule in the mammalian cell.

In a further aspect, the invention features a cell comprising a nucleic acid molecule encoding a protein molecule shown in SEQ ID NO. 1. The invention also features cells comprising a nucleic acid molecule encoding a protein molecule whose function is to protect cells against degeneration and/or cell death and whose amino acid sequence comprises the sequence shown in SEQ ID NO. 1 or a functional variant thereof. It also features cells comprising a D N A molecule capable of hybridizing with the complement of the c D N A described in SEQ ID NO. 2 under stringent conditions. In preferred embodiments, said cell is a bacterial cell, a yeast cell, a mammalian cell, or a cell of an insect. In particular, the invention features a bacterial cell comprising a plasmid adapted for expression in a bacterial cell, said plasmid comprising a nucleic acid molecule which encodes a protein molecule shown in SEQ ID NO. 1, and the regulatory elements necessary for expression of said molecule in the bacterial cell. The invention also features a yeast cell comprising a plasmid adapted for expression in a yeast cell, said plasmid comprises a nucleic acid molecule encoding a protein molecule shown in SEQ ID NO. 1, and the regulatory elements necessary for expression of said molecule in the yeast cell. It further features a mammalian cell comprising a plasmid adapted for expression in a mammalian cell, said plasmid comprising a nucleic acid molecule which encodes a protein molecule shown in SEQ ID NO.1, and the regulatory elements necessary for expression of said molecule in the mammalian cell.

The invention further features an antibody specifically immunoreactive with an immunogen, wherein said immunogen is shown in SEQ ID NO. 1 or wherein said immunogen is a protein molecule, the function of which is to protect cells against degeneration and/or cell death, wherein the amino acid sequence of the protein molecule comprises the sequence shown in SEQ ID NO. 1 or a functional variant thereof. In another aspect, the invention aims at an in-vitro method of detecting pathological cells in a subject which comprises immunocytochemically staining cells with the aforementioned antibody, wherein a low degree of staining in said cell compared to a reference cell representing a known health status indicates a pathological change of said cell. The invention is particularly suited to detect pathological structures in the brain of a subject - the detection method comprises immunocytochemically staining said pathological structures with said antibody. It is also especially suited to detect pathological cells of the muscular system, prostate, stomach, testis, ovary, adrenal glands, mammary glands, liver, spleen, lung, trachea or placenta.

In another aspect, the invention features an in-vitro method of diagnosing or prognosing a disease, in particular a neurological disease, in a subject comprising:
determining a level, or an activity, or both said level and said activity, of at least one substance which is selected from the group consisting of
   (a) a D N A molecule encoding a protein molecule, wherein the amino acid sequence of the protein molecule comprises the sequence shown in SEQ ID NO.1 or a functional variant thereof,
   (b) a transcription product of a D N A molecule encoding a protein molecule, wherein the amino acid sequence of the protein molecule comprises the sequence shown in SEQ ID NO.1 or a functional variant thereof,
   (c) a protein molecule wherein the amino acid sequence of the protein molecule comprises the sequence shown in SEQ ID NO.1 or a functional variant thereof,
   (d) a D N A molecule capable of hybridizing with the complement of the c D N A described in SEQ ID NO. 2 under stringent conditions,
   (e) a transcription product of a D N A molecule, wherein said D N A molecule is capable of hybridizing with the complement of the c D N A described in SEQ ID NO. 2 under stringent conditions,
   (f) a translation product of a D N A molecule, wherein said D N A molecule is capable of hybridizing with the complement of the c D N A described in SEQ ID NO. 2 under stringent conditions,
   (g) a molecule affecting a level, or an activity, or both said level and said activity, of at least one substance which is selected from the group consisting of (a) to (f),
   (h) a molecule which is affected in its level, or its activity, or both its level and activity, by at least one substance which is selected from the group consisting of (a) to (f),

   and comparing said level, or said activity, or both said level and said activity, of at least one of said substances (a) to (h) to a reference value representing a known disease or health status, thereby diagnosing or prognosing a disease, in particular a neurological disease, in said subject.

In another aspect, the invention features an in-vitro method of monitoring the progression of a disease, in particular a neurological disease, in a subject, comprising:
determining a level, or an activity, or both said level and said activity, of at least one substance which is selected from the group consisting of
   (a) a D N A molecule encoding a protein molecule, wherein the amino acid sequence of the protein molecule comprises the sequence shown in SEQ ID NO.1 or a functional variant thereof,
   (b) a transcription product of a D N A molecule encoding a protein molecule, wherein the amino acid sequence of the protein molecule comprises the sequence shown in SEQ ID NO.1 or a functional variant thereof,
   (c) a protein molecule, wherein the amino acid sequence of the protein molecule comprises the sequence shown in SEQ ID NO.1 or a functional variant thereof,
   (d) a D N A molecule capable of hybridizing with the complement of the c D N A described in SEQ ID NO. 2 under stringent conditions,
   (e) a transcription product of a D N A molecule, wherein said D N A molecule is capable of hybridizing with the complement of the c D N A described in SEQ ID NO. 2 under stringent conditions,
   (f) a translation product of a D N A molecule, wherein said D N A molecule is capable of hybridizing with the complement of the c D N A described in SEQ ID NO. 2 under stringent conditions,
   (g) a molecule affecting a level, or an activity, or both said level and said activity, of at least one substance which is selected from the group consisting of (a) to (f),
   (h) a molecule which is affected in its level, or its activity, or both its level and activity, by at least one substance which is selected from the group consisting of (a) to (f),

   and comparing said level, or said activity, or both said level and said activity, of at least one of said substances (a) to (h) to a reference value representing a known disease or health status, thereby monitoring progression of a disease, in particular a neurological disease, in said subject.

In still a further aspect, the invention features an in-vitro method of evaluating a treatment for a disease, in particular a neurological disease, in a subject, said method comprising:
determining a level, or an activity, or both said level and said activity, of at least one substance which is selected from the group consisting of
   (a) a D N A molecule encoding a protein molecule, wherein the amino acid sequence of the protein molecule comprises the sequence shown in SEQ ID NO.1 or a functional variant thereof,
   (b) a transcription product of a D N A molecule encoding a protein molecule, wherein the amino acid sequence of the protein molecule comprises the sequence shown in SEQ ID NO.1 or a functional variant thereof,
   (c) a protein molecule, wherein the amino acid sequence of the protein molecule comprises the sequence shown in SEQ ID NO.1 or a functional variant thereof,
   (d) a D N A molecule capable of hybridizing with the complement of the c D N A described in SEQ ID NO. 2 under stringent conditions,
   (e) a transcription product of a D N A molecule, wherein said D N A molecule is capable of hybridizing with the complement of the c D N A described in SEQ ID NO. 2 under stringent conditions,
   (f) a translation product of a D N A molecule, wherein said D N A molecule is capable of hybridizing with the complement of the c D N A described in SEQ ID NO. 2 under stringent conditions,
   (g) a molecule affecting a level, or an activity, or both said level and said activity, of at least one substance which is selected from the group consisting of (a) to (f),
   (h) a molecule which is affected in its level, or its activity, or both its level and activity, by at least one substance which is selected from the group consisting of (a) to (f),

   and comparing said level, or said activity, or both said level and said activity, of at least one of said substances (a) to (h) to a reference value representing a known disease or health status, thereby evaluating a treatment for a disease, in particular a neurological disease, in said subject.

In a further aspect, the invention features a kit for diagnosis, or prognosis of a disease, said kit comprising:
(1) at least one reagent which is selected from the group consisting of reagents that selectively detect
   (a) a D N A molecule encoding a protein molecule, wherein the amino acid sequence of the protein molecule comprises the sequence shown in SEQ ID NO.1 or a functional variant thereof,
   (b) a transcription product of a D N A molecule encoding a protein molecule, wherein the amino acid sequence of the protein molecule comprises the sequence shown in SEQ ID NO.1 or a functional variant thereof,
   (c) a protein molecule, wherein the amino acid sequence of the protein molecule comprises the sequence shown in SEQ ID NO.1 or a functional variant thereof,
   (d) a D N A molecule capable of hybridizing with the complement of the c D N A described in SEQ ID NO. 2 under stringent conditions,
   (e) a transcription product of a D N A molecule, wherein said D N A molecule is capable of hybridizing with the complement of the c D N A described in SEQ ID NO. 2 under stringent conditions,
   (f) a translation product of a D N A molecule, wherein said D N A molecule is capable of hybridizing with the complement of the c D N A described in SEQ ID NO. 2 under stringent conditions,
   (g) a molecule affecting a level, or an activity, or both said level and said activity, of at least one substance which is selected from the group consisting of (a) to (f),
   (h) a molecule which is affected in its level, or its activity, or both its level and activity, by at least one substance which is selected from the group consisting of (a) to (f),
(2) instructions for diagnosing, or prognosing said disease by
   (i) detecting a level, or an activity, or both said level and said activity, of at least one substance which is selected from the group consisting of (a) to (h) in a sample from said subject;
      and
   (ii) diagnosing, or prognosing said disease, wherein a varied level, or activity, or both said level and said activity, of at least one substance which is selected from the group consisting of (a) to (h) compared to a reference value representing a known health status;
      or a level, or activity, or both said level and said activity, of at least one substance which is selected from the group consisting of (a) to (h) similar or equal to a reference value representing a known disease status indicates diagnosis, or prognosis of said disease.

In a further aspect, the kit may be used in monitoring success or failure of a therapeutic treatment of said subject. It can also be used in monitoring the progression of a disease.

Preferred embodiments of the above mentioned methods and kit of diagnosing or prognosing diseases, or monitoring the progression thereof, or evaluating a treatment thereof, are now disclosed in detail.

In a preferred embodiment, the function of said protein molecule or a functional variant thereof is to protect cells from degeneration and/or cell death.

In another preferred embodiment, said D N A molecule capable of hybridizing with the complement of the c D N A described in SEQ ID NO. 2 encodes a protein molecule, the function of which is to protect cells against cell degeneration and/or cell death.

In preferred embodiments, said subjects suffer from Alzheimer's disease and related neurofibrillary disorders, or degenerative states, e.g. neurodegenerative states, characterized by cell degeneration or cell death. Further examples of neurological diseases are Parkinson's disease, Huntington disease, amyotrophic lateralsclerosis and Pick's disease.

It is particularly preferred that said sample is a brain tissue or other body cells including cells of the muscular system, prostate, stomach, testis, ovary, adrenal glands, mammary glands, liver, spleen, lung, trachea, or placenta. The sample might also be cerebrospinal fluid or another body fluid.

According to the present invention, a reduction in the level, or activity, or both said level and said activity, of (i) a transcription product of a D N A molecule encoding a protein molecule, whose amino acid sequence comprises the sequence shown in SEQ ID NO.1 or a functional variant thereof or (ii) a protein molecule whose amino acid sequence comprises the sequence shown in SEQ ID NO. 1 or a functional variant thereof, in a sample from said subject relative to a reference value representing a known health status indicates the presence of a pathological status in said subject. In particular, a reduction in the level, or activity, or both said level and said activity of SELADIN-1 or *SELADIN-1* transcripts in said subject's brain regions affected heavily by neurodegeneration relative to a reference value representing a known health status indicates a diagnosis or prognosis of Alzheimer's disease. Predominantly neurons within the inferior temporal lobe, the entorhinal cortex, the hippocampus and the amygdala degenerate in Alzheimer's disease.

It might be preferred that said subject has previously been determined to have one or more factors indicating that such subject is afflicted with a disease under study, in particular a neurological disease.

In preferred embodiments, said subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In preferred embodiments, at least one of said substances is detected using an immunoassay, an enzyme activity assay and/or a binding assay.

In preferred embodiments, measurement of the level of transcription products of the *SELADIN-1* gene, or a functional variant thereof, is performed in body cells using Northern blots with probes specific for the *SELADIN-1* gene or said variant. Quantitative PCR with primer combinations to amplify *SELADIN-1* gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed. W.H. Freeman and Company, 1992).

In preferred embodiments, said level or activity of the protein molecule shown in SEQ ID NO. 1, or a functional variant or fragment thereof, is detected using an immunoassay. These assays can measure the amount of binding between said protein molecule and an anti-protein antibody, e.g. an anti-SELADIN-1 antibody, by the use of enzymatic, chromodynamic, radioactive, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect SELADIN-1 or a functional variant or fragment thereof. It is preferred that it does not substantially interact with any other protein present in said sample.

Monoclonal antibodies capable of recognizing a protein molecule of SEQ ID NO. 1 or a functional variant or fragment thereof can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; ; Watson et al., Rekombinierte DNA, 2nd ed., Spektrum Akademischer Verlag GmbH, 1993; Watson et al, Recombinant DNA, 2nd ed., W. H. Freeman and Company, 1992; the contents of which are incorporated herein by reference). Monoclonal antibodies useful in the methods of the invention are directed to an epitope of SELADIN-1 or a functional variant or fragment thereof, such that the complex formed between the antibody and SELADIN-1, or between the antibody and said functional variant or fragment, can be recognized in detection assays. The term "antibodies" encompasses all forms of antibodies known in the art, such as polyclonal, monoclonal, chimeric, recombinatorial, single chain antibodies as well as fragments thereof which specifically bind to SELADIN-1, or to a functional variant or fragment thereof.

Antibodies or ligands might also be used in detecting specifically molecules mentioned in the above described methods and kit under g) and h) above.

If luminescent labels are used in any detection assay, it is preferred to use a confocal optical set-up.

In further preferred embodiments, said reference value is that of a level, or an activity, or both said level and said activity, of at least one substance which is selected from the group consisting of (a) to (h) described above in a sample from a subject not suffering of the disease under study, in particular a neurological disease such as Alzheimer's disease. The healthy subject can be of the same weight, age, and gender as the subject who is being diagnosed or prognosed for said disease. In some cases, it might be preferred to use a reference value from the subject which is diagnosed.

In a preferred embodiment, the level, or the activity, or both said level and said activity, of at least one of said substances (a) to (h) described above in a sample is determined at least twice, e.g. at two points which are weeks or months apart. The levels or activities at these two time points are compared in order to monitor the progression of said disease. It might be preferred to take a series of samples over a period of time. In further preferred embodiments, said subject receives a treatment prior to one or more of said sample gatherings.

The invention can be employed in a method of treating or preventing a disease, in particular a neurological disease, in a subject comprising administering to said subject in a therapeutically effective amount an agent or agents which affect a level, or an activity, or both said level and said activity, of at least one substance which is selected from the group consisting of
(a) a D N A molecule encoding a protein molecule, wherein the amino acid sequence of the protein molecule comprises the sequence shown in SEQ ID NO.1 or a functional variant thereof,
(b) a transcription product of a D N A molecule encoding a protein molecule, wherein the amino acid sequence of the protein molecule comprises the sequence shown in SEQ ID NO.1 or a functional variant thereof,
(c) a protein molecule, wherein the amino acid sequence of the protein molecule comprises the sequence shown in SEQ ID NO.1 or a functional variant thereof,
(d) a D N A molecule capable of hybridizing with the complement of the c D N A described in SEQ ID NO. 2 under stringent conditions,
(e) a transcription product of a D N A molecule, wherein said D N A molecule is capable of hybridizing with the complement of the c D N A described in SEQ ID NO. 2 under stringent conditions,
(f) a translation product of a D N A molecule, wherein said D N A molecule is capable of hybridizing with the complement of the c D N A described in SEQ ID NO. 2 under stringent conditions,
(g) a molecule affecting a level, or an activity, or both said level and said activity, of at least one substance which is selected from the group consisting of (a) to (f),
(h) a molecule which is affected in its level, or its activity, or both its level and activity, by at least one substance which is selected from the group consisting of (a) to (f).

In a preferred embodiment, the function of said protein molecule or a functional variant thereof is to protect cells from degeneration and/or cell death.

In another preferred embodiment, said D N A molecule capable of hybridizing with the complement of the c D N A described in SEQ ID NO. 2 encodes a protein molecule, the function of which is to protect cells against cell degeneration and/or cell death.

In preferred embodiments, said subjects suffer from Alzheimer's disease and related neurofibrillary disorders, or degenerative states, such as neurodegenerative states, characterized by cell degeneration or cell death. Further examples of neurological diseases are Parkinson's disease, Huntington disease, amyotrophic lateralsclerosis and Pick's disease.

In preferred embodiments, the method comprises the application of per se known methods of gene therapy nucleic acid technology to administer said agent or said agents.

In general, gene therapy includes several approaches: molecular replacement of a mutated gene, addition of a new gene resulting in the synthesis of a therapeutic protein, and modulation of endogeneous cellular gene expression by recombinant expression methods or by drugs. Gene-transfer techniques are described in detail (see e.g. Behr, Acc. Chem. Res. 26, 274 - 278, 1993; Mulligan, Science 260, 926 - 931, 1993; the contents of which are incorporated herein by reference) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane perturbation by chemical (solvents, detergents, polymers, enzymes) or physical means (mechanic, osmotic, thermic, electric shocks). The postnatal gene transfer into the central nervous system has been described in detail (see e.g. Wolff, Current Opinion in Neurobiology, 3, 743 - 748, 1993; the contents of which are incorporated herein by reference).

In preferred embodiments, the method comprises grafting donor cells into the central nervous system, preferably the brain, of said subject, said subject or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phospate transfection, liposomal mediated transfection, etc.

In preferred embodiments, said agent is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

In preferred embodiments, the therapeutic nucleic acid or protein reduces or prevents the degeneration of cells, in particular neurons and slows brain amyloid formation.

In a further aspect, the invention features a method for identifying an agent that affects an activity, or level, or both said activity or level, of at least one substance which is selected from the group consisting of
(a) a D N A molecule encoding a protein molecule, wherein the amino acid sequence of the protein molecule comprises the sequence shown in SEQ ID NO.1 or a functional variant thereof,
(b) a transcription product of a D N A molecule encoding a protein molecule, wherein the amino acid sequence of the protein molecule comprises the sequence shown in SEQ ID NO.1 or a functional variant thereof,
(c) a protein molecule, wherein the amino acid sequence of the protein molecule comprises the sequence shown in SEQ ID NO.1 or a functional variant thereof,
(d) a D N A molecule capable of hybridizing with the complement of the c D N A described in SEQ ID NO. 2 under stringent conditions,
(e) a transcription product of a D N A molecule, wherein said D N A molecule is capable of hybridizing with the complement of the c D N A described in SEQ ID NO. 2 under stringent conditions,
(f) a translation product of a D N A molecule, wherein said D N A molecule is capable of hybridizing with the complement of the c D N A described in SEQ ID NO. 2 under stringent conditions,

comprising the steps of:
(i) providing a sample containing at least one substance which is selected from the group consisting of (a) to (f),
(ii) contacting said sample with at least one agent,
(iii) comparing an activity, or level, or both said activity and level, of at least one of said substances before and after contacting.

Preferably, the function of said protein molecule or a variant thereof is to protect cells from degeneration and/or cell death. Preferably, said D N A molecule capable of hybridizing with the complement of the c D N A described in SEQ ID NO. 2 encodes a protein molecule, whose function is to protect cells against degeneration and/or cell death.

Other features and advantages of the invention will be apparent from the following detailed description of the figures, the examples and the claims.

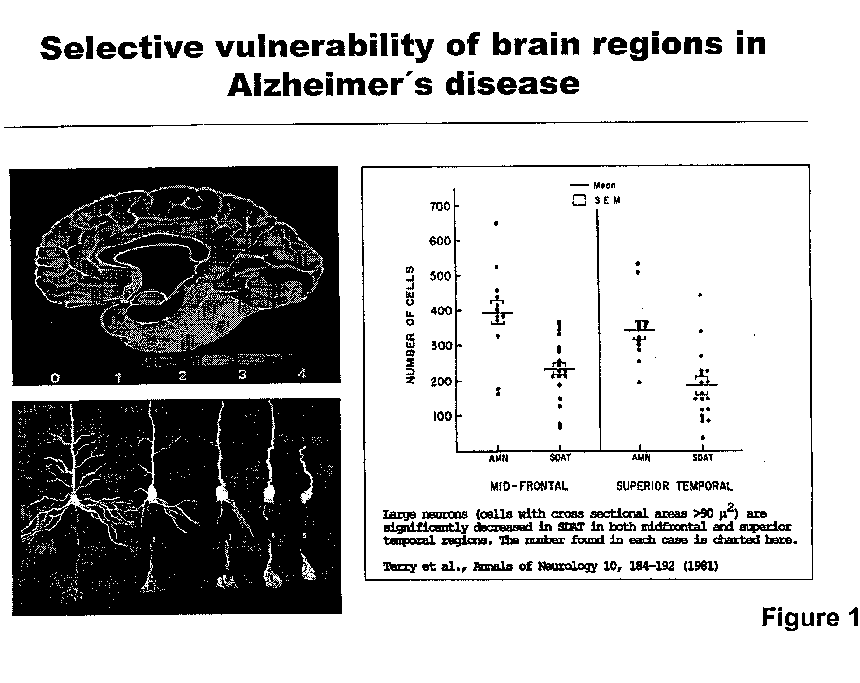

Figure 1 depicts the selective vulnerability of brain regions in Alzheimer's disease. Predominantly neurons within the inferior temporal lobe, the entorhinal cortex, the hippocampus and the amygdala degenerate in Alzheimer's disease (Terry et al., Annals of Neurology, 10, 184-192, 1981). These brain regions are predominantly involved in the processing of learning and memory functions. In contrast, neurons within the frontal cortex, the occipital cortex and the cerebellum are largely intact and preserved from the neurodegenerative process in Alzheimer's disease.

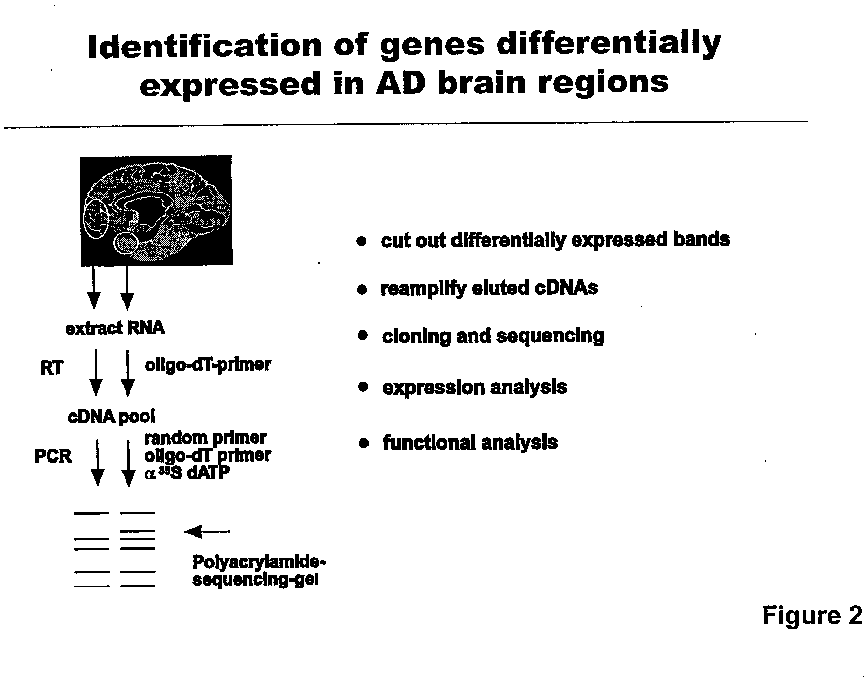

Figure 2 discloses the identification of genes differentially expressed in brain regions from Alzheimer's disease patients. Brain areas with massive neuronal cell loss as well as areas with largely preserved neurons were identified and RNA extracted. Synthesis of cDNA was performed using an oligo-dT primer followed by PCR using the oligo-dT primer in combination with random primers and (α³⁵S)-dATP. Reactions were separated on DNA sequencing gels, DNA bands visualized by autoradiography and bands lighting up in different intensities were cut out. DNA fragments were reamplified by PCR, cloned in *E. coli* and sequences determined. Expression and functional analyses were performed.

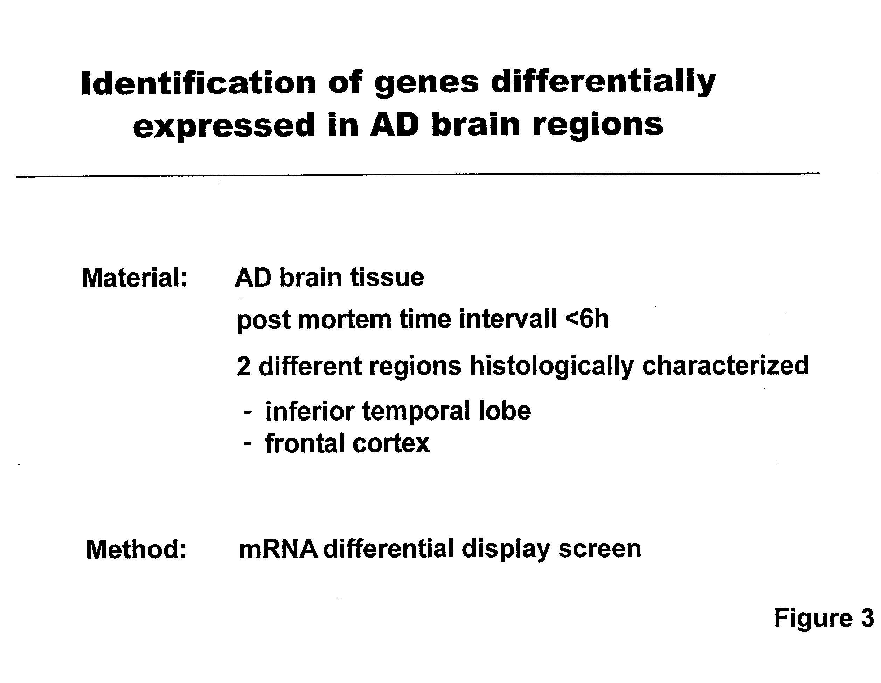

Figure 3 depicts the specifications of Alzheimer's disease brain tissue as it was used in the examples. Brain tissues from Alzheimer's disease patients and control subjects were removed within 6 hours of death, and immediately frozen on dry ice. For RNA extraction tissue sections from the inferior temporal lobe and frontal cortex were chosen.

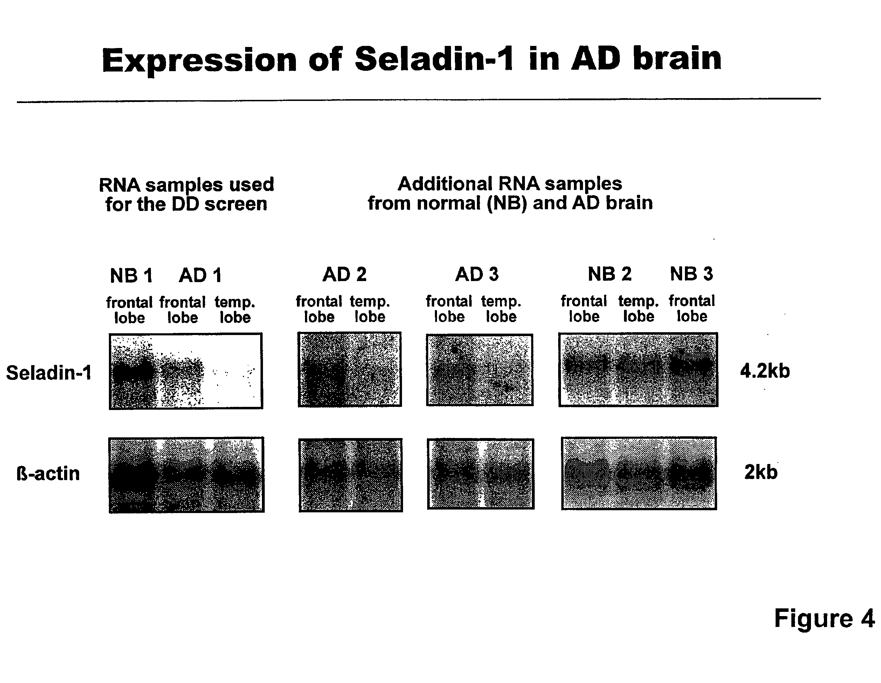

Figure 4 discloses the quantification of *SELADIN-1* transcripts in brain tissue from Alzheimer's disease and control subjects by Northern blot analyses. Transcript levels were significantly lower in brain regions with severe neurodegeneration, i. e. temporal lobe in Alzheimer's disease (AD1-3) but not in normal brain (NB1-3), as compared to protected brain regions, i. e. frontal lobe. This decrease was specific as indicated by unchanged β-actin transcript levels used to control for equal loading of RNA.

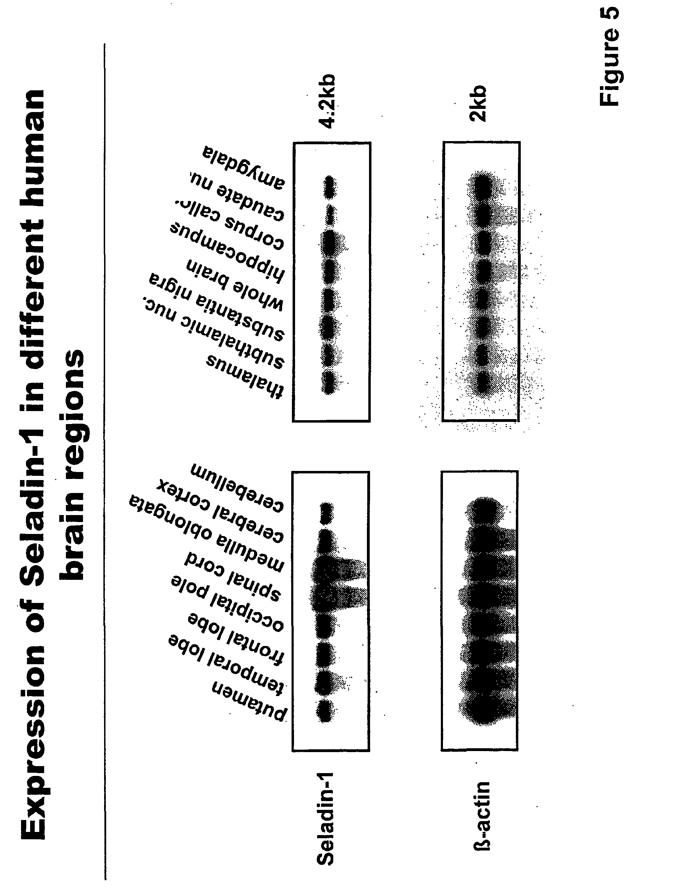

Figure 5 depicts the transcription levels of the *SELADIN-1* gene in different human brain regions. The *SELADIN-1* gene was found to be expressed throughout the human brain. In particular transcription levels are high in all cortical areas, the hippocampus, the amygdala, the spinal cord, and the medulla. Note the unchanged levels in temporal lobe versus frontal lobe in this brain derived from a cognitively normal control subject without any signs of Alzheimer's disease. The analysis of β-actin transcripts was used as loading control.

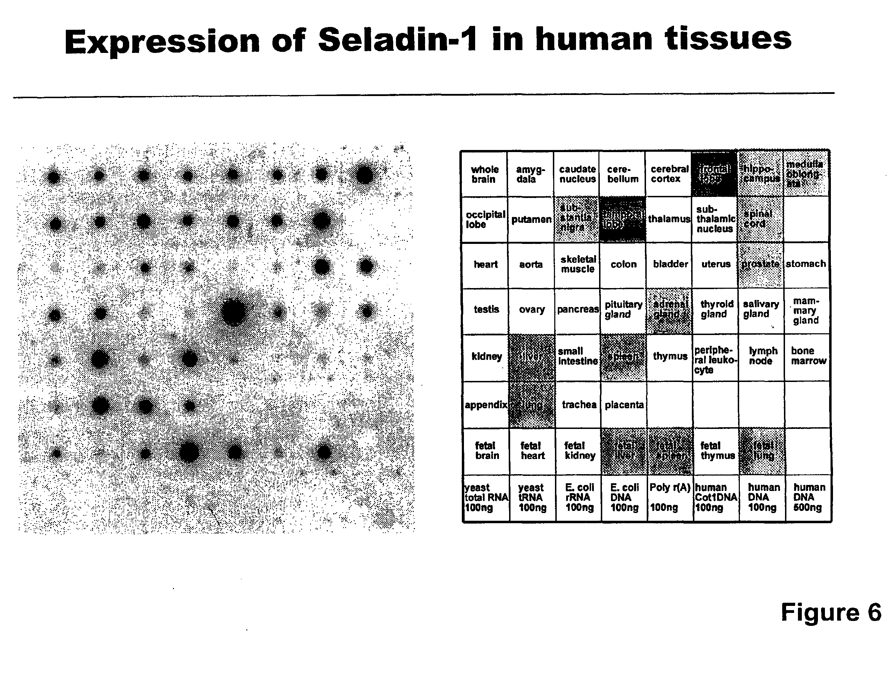

Figure 6 depicts the distribution of *SELADIN-1* transcripts in human tissues. Comparable samples of RNA were spotted on nitrocellulose filters and *SELADIN-1* transcripts were quantified by hybridization using a labeled SELADIN-1 gene specific probe. Significant levels of *SELADIN-1* gene transcripts were found in all brain regions tested. Transcripts were also detected in other tissues, however, strong variations in signal intensity indicated a tissue specific regulation of *SELADIN-1* expression.

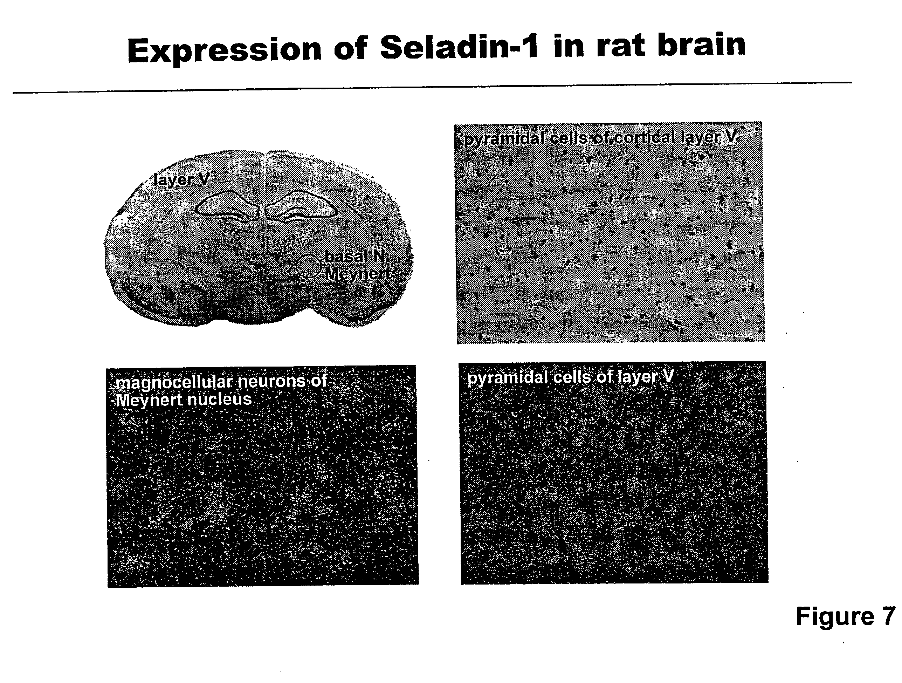

Figure 7 depicts the expression of the *SELADIN-1* gene in rat brain cortex, hippocampus and basal nucleus analyzed by in situ hybridization. This staining pattern along with the higher magnifications indicate that *SELADIN-1* is predominantly expressed in neurons. No significant hybridization signals were observed with glial cells.

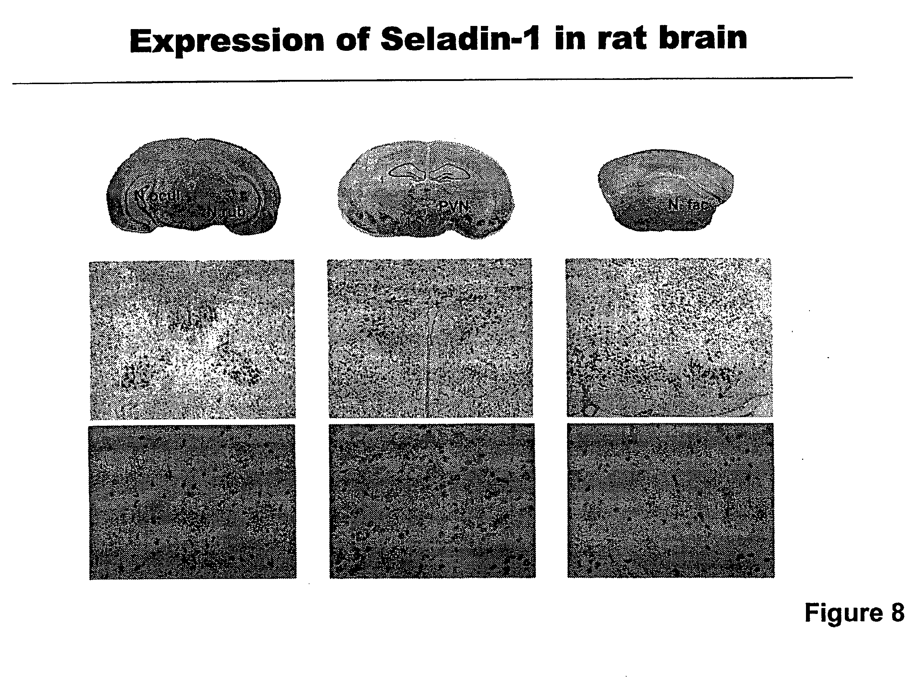

Figure 8 depicts the expression of *SELADIN-1* in rat brain nuclei. Strong *SELADIN-1* expression was found in the occulomotor, paraventricular, red and facial nuclei. Higer magnifications indicate predominant hybridization with neurons. No significant hybridization signals were observed with glial cells.

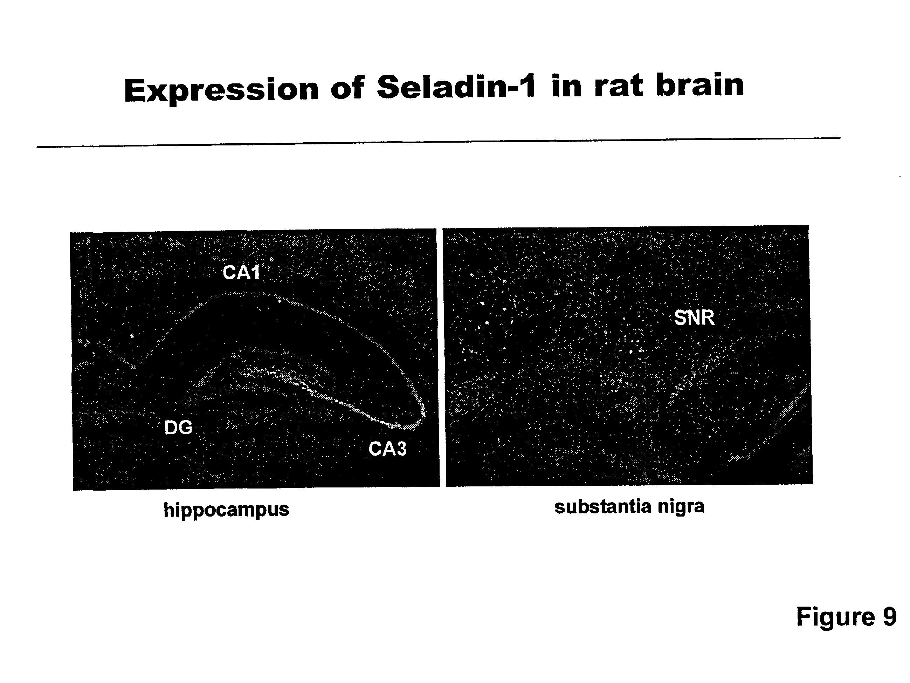

Figure 9 depicts the expression of *SELADIN-1* in rat brain hippocampus and substantia nigra. In situ hybridization with *SELADIN-1* transcripts was detected by photoemulsionautoradiography, confirming the neuron specific expression of this gene.

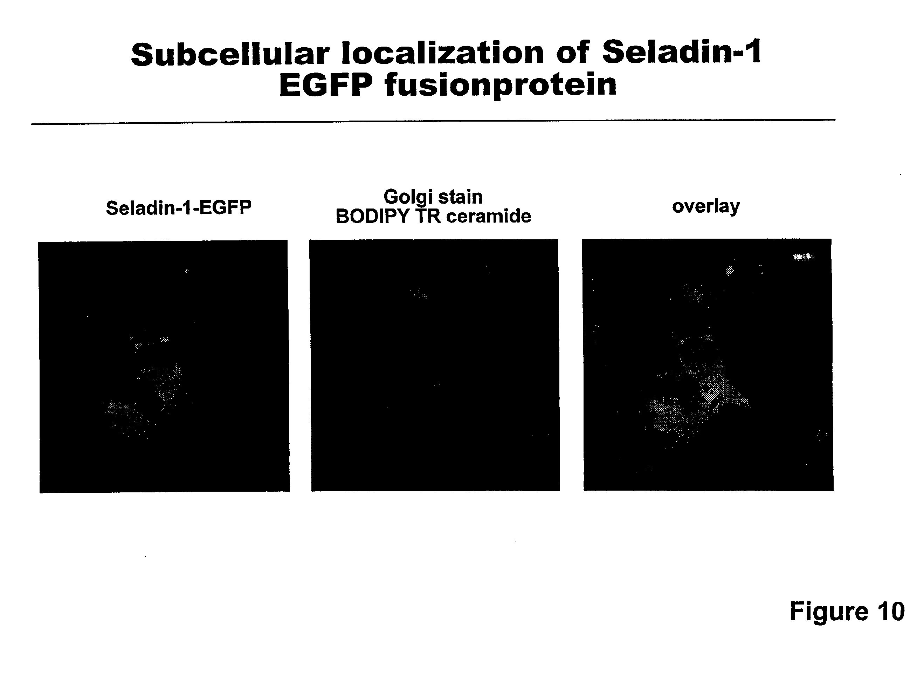

Figure 10 discloses the subcellular localization of a SELADIN-1-EGFP (enhanced green fluorescent protein) fusion in transfected cos cells. The confocal micrographs show the co-localization of the SELADIN-1-EGFP fusion with the golgi specific stain BODIPY TR ceramide indicating localization of SELADIN-1 in the Golgi apparatus and the endoplasmic reticulum.

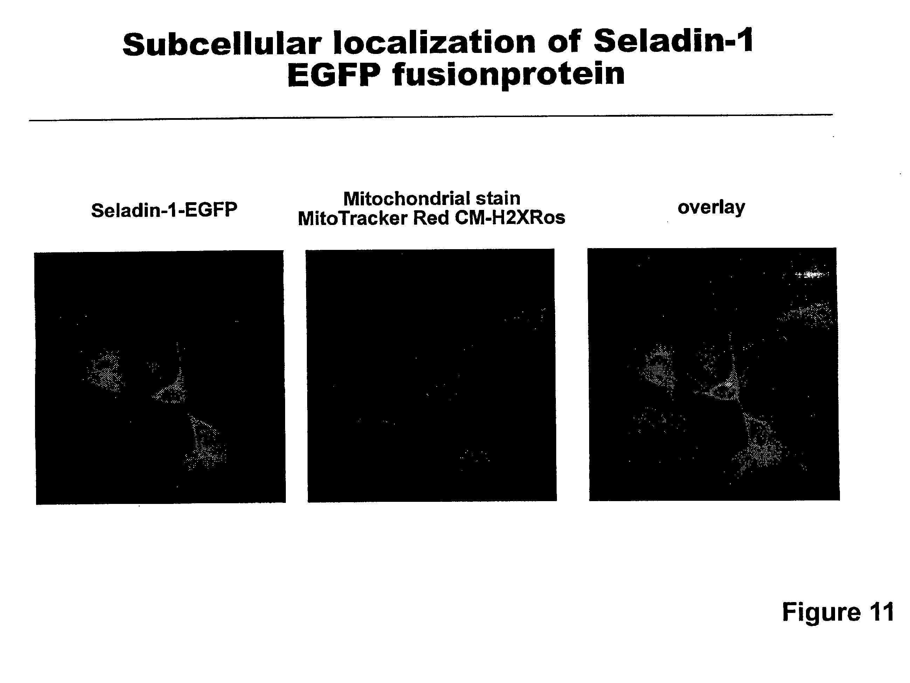

Figure 11 discloses that the SELADIN-1-EGFP fusion does not localize to mitochondria in transfected cos cells in spite of a putative mitochondrial targeting sequence close to the N-terminus of the SELADIN-1 protein. The confocal micrographs show the different staining patterns caused by the SELADIN-1-EGFP fusion and the specific mitochondrial stain Mito Tracker Red CM-H₂XRos.

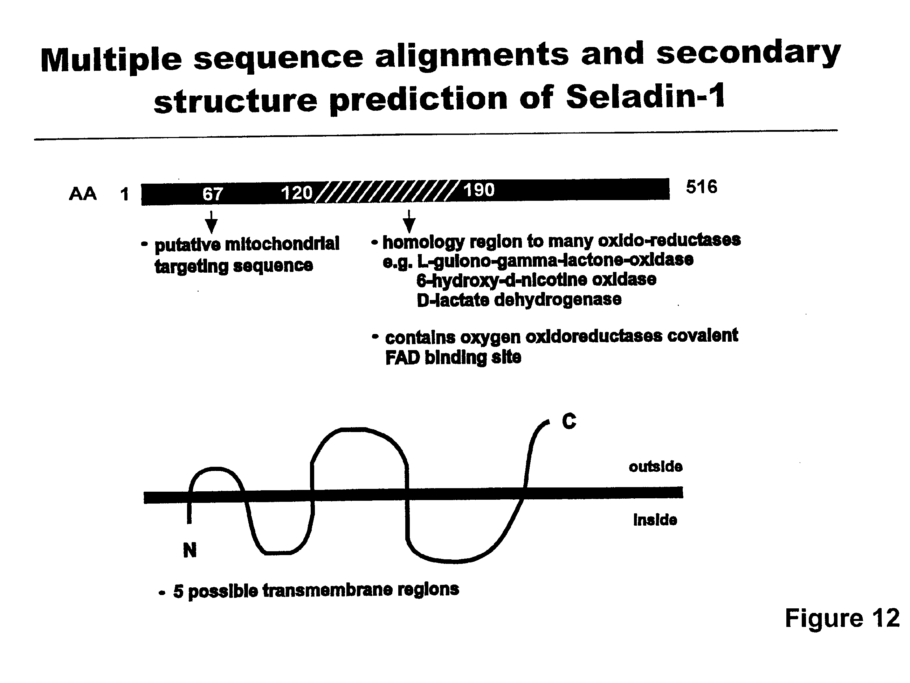

Figure 12 discloses structural features of the SELADIN-1 protein based on multiple sequence alignments and secondary structure predictions. Near the N-terminus the SELADIN-1 protein contains a putative mitochondrial localization signal that appears to be inactive in transfected cos cells or when used in EGFP fusions. The central region of the protein contains a sequence that is homologous to a family of oxidoreductases and that contains a FAD site for covalent binding. The protein is predicted to contain five transmembrane regions. The expression in neurons, the co-localization in the Golgi apparatus and the endoplasmic reticulum of the SELADIN-1 protein, the amyloid precursor protein (APP) and the presenilins PS1 and PS2 and furthermore the transmembrane character suggest a functional relationship between these proteins. Mutations in both APP and presenilins were shown to cause an increase in the production of β-amyloid. In a similar way the SELADIN-1 protein might be involved in common biological pathways influencing the processing of the amyloid precursor protein and the generation of Aβ. Using the SELADIN-1 protein as a probe, interaction partners can be identified which might represent new AD drug targets.

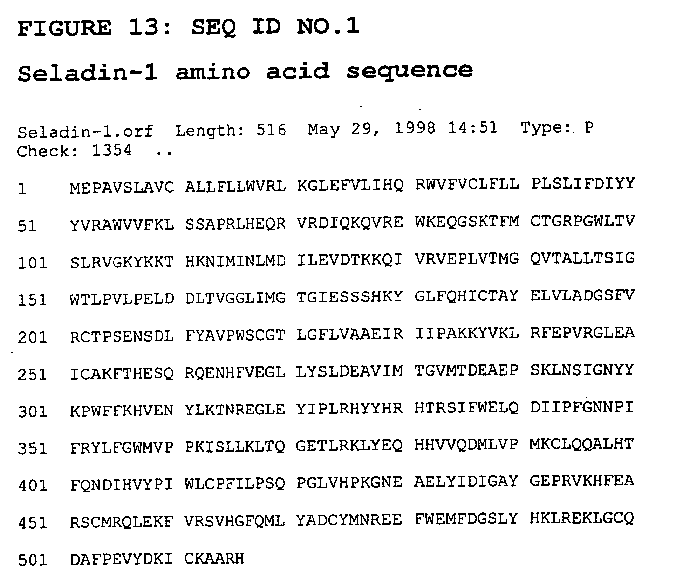

Figure 13 discloses the protein sequence of SELADIN-1 (SEQ ID NO. 1). The full length protein consists of 516 amino acid residues. The sequence is given in the one letter amino acid code.

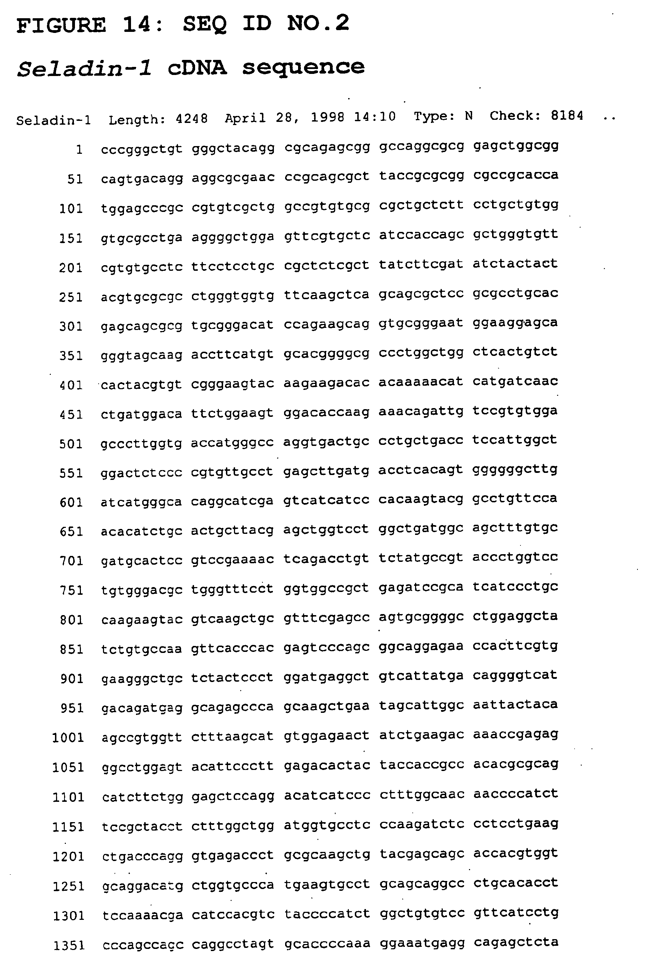

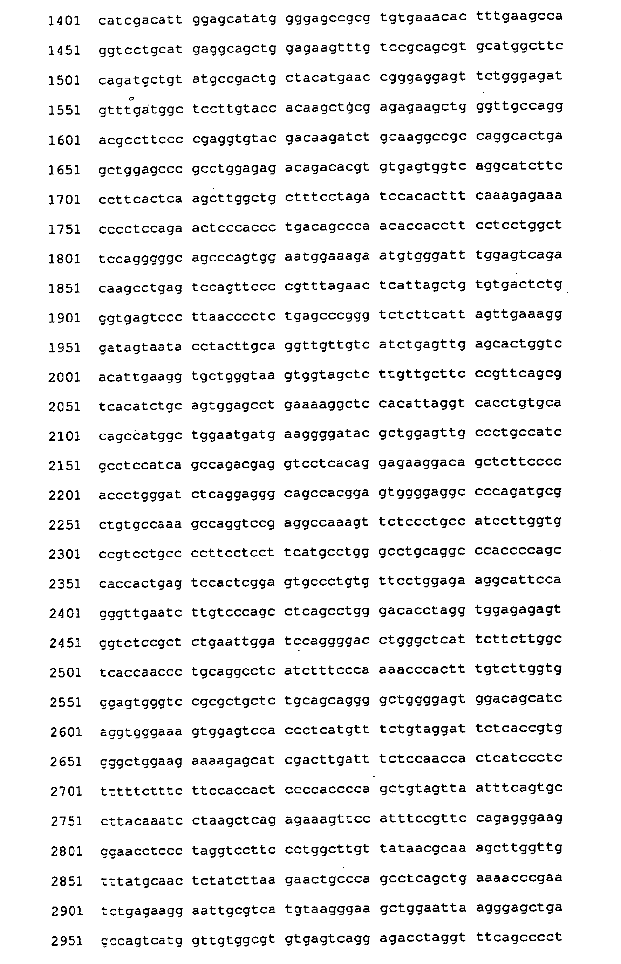

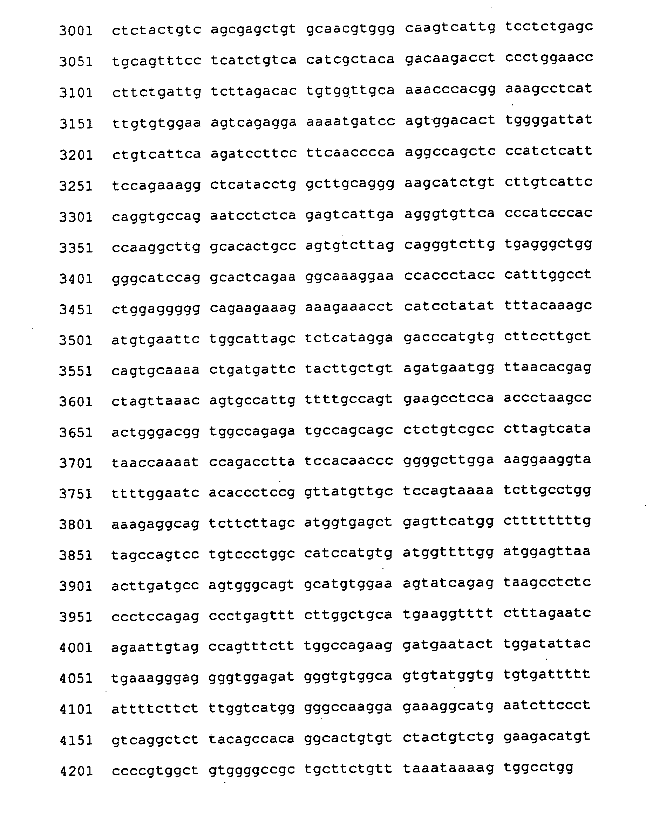

Figure 14 discloses the nucleotide sequence of the cloned *SELADIN-1* cDNA (SEQ ID NO. 2) comprising 4248 nucleotides. The coding sequence for the SELADIN-1 protein starts at nucleotide position 100 and stops at position 1648.

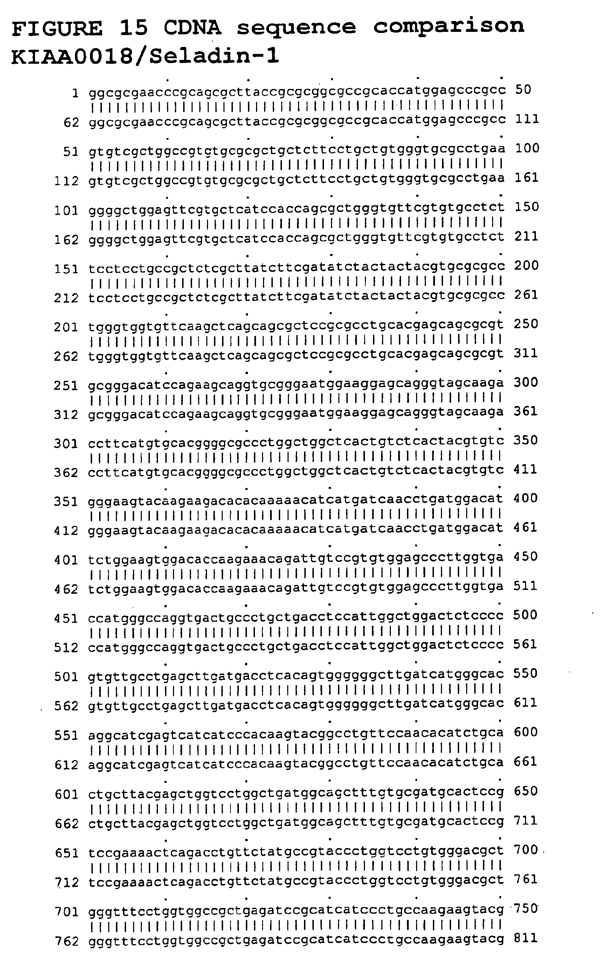

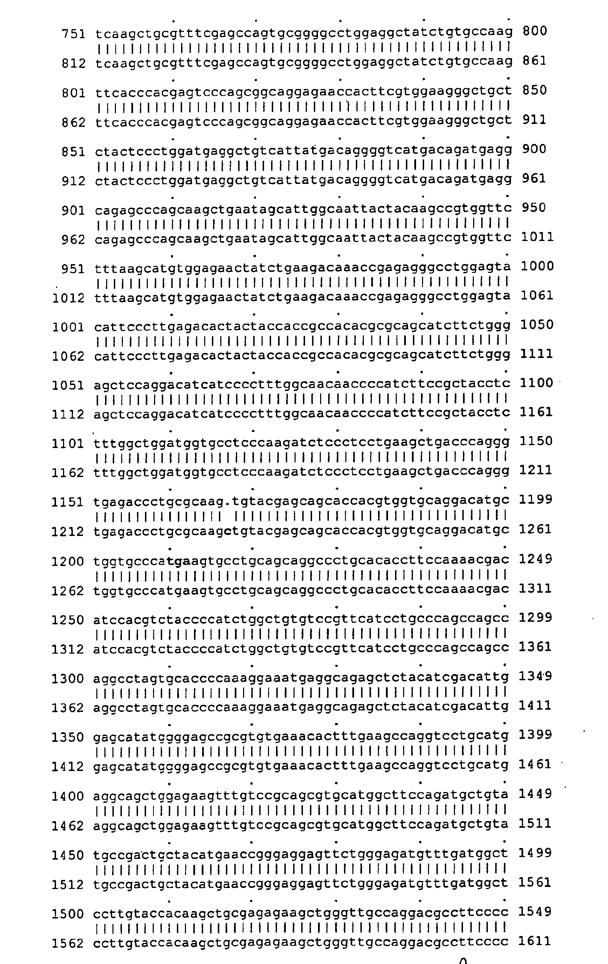

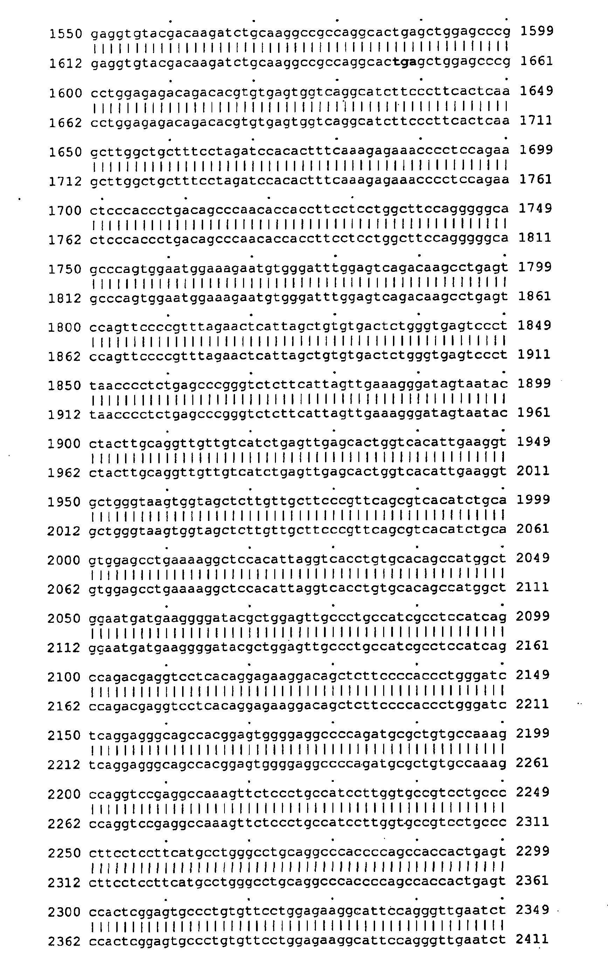

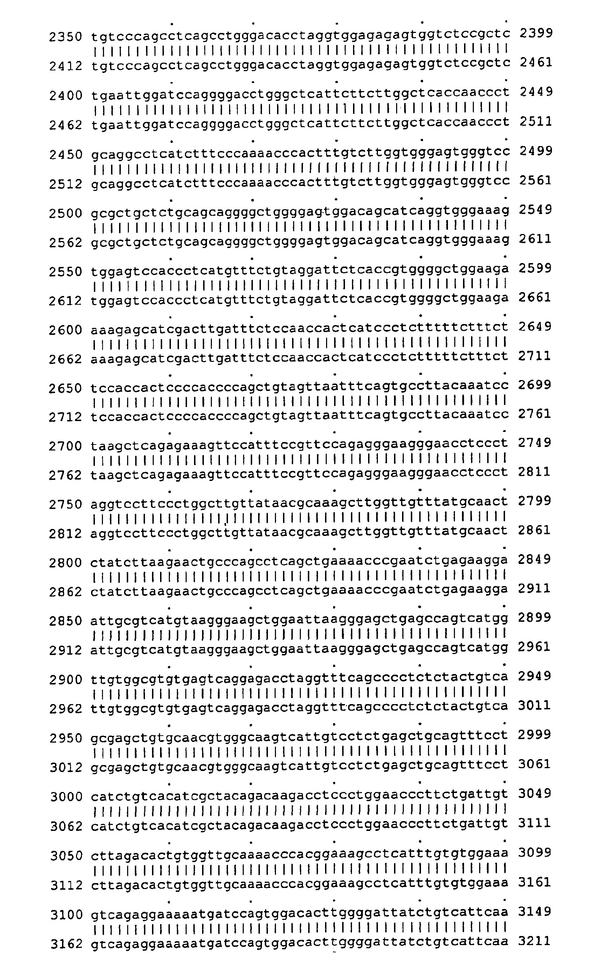

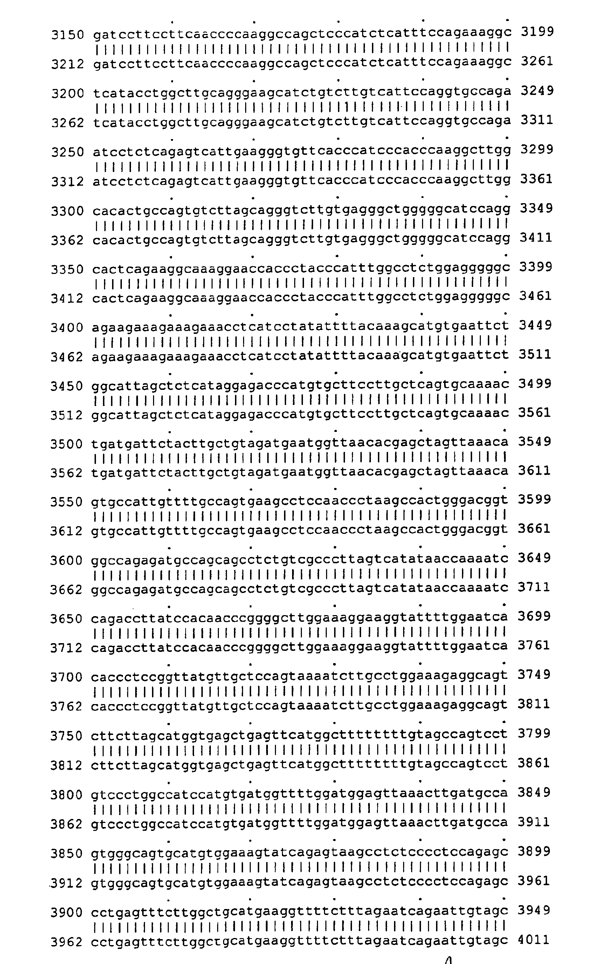

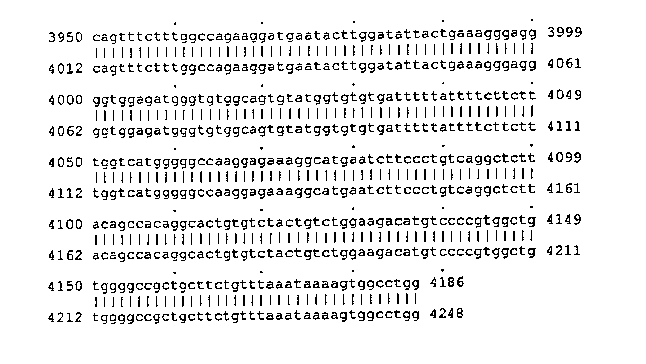

Figure 15 discloses the comparison of nucleotide sequences of the cloned *SELADIN-1* cDNA comprising 4248 nucleotides and the KIAA0018 cDNA comprising 4186 nucleotides. A significant difference exists at position 1228 of the *SELADIN-1* sequence where a C nucleotide (C/G basepair) is missing in the *KIAA0018* sequence. This results in a frameshift in the open reading frame in the *KIAA0018* sequence relative to the *SELADIN-1* sequence. The consequence is that the translation product of the *KIAA0018* gene is 390 amino acids in length compared to 516 amino acid residues of the SELADIN-1 translation product. In addition to the difference in length, the frameshift causes a difference between the C-terminal 14 amino acids of the KIAA0018 protein and the corresponding sequence area of the SELADIN-1 polypeptide (pos. 377 - 390). The coding sequence for the SELADIN-1 protein starts at nucleotide position 100 and stops at position 1648.

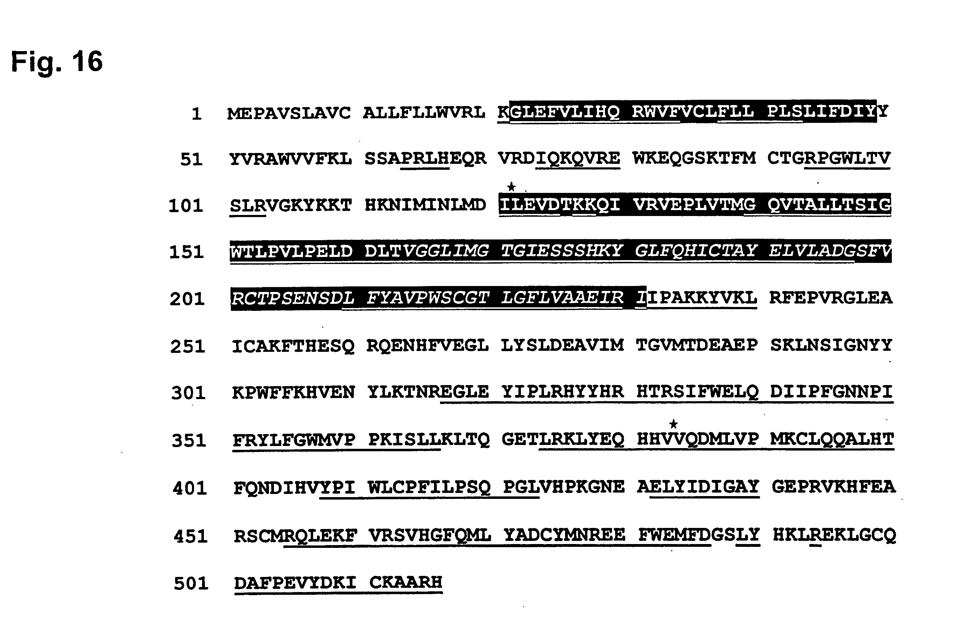

Figure 16 shows the amino acid sequence of SELADIN-1. A differential display approach (von der Kammer, H. et al., Nucleic acid research, 27, 2211, 1999; von der Kammer, H. et al., J. Biol. Chem. 273, 14538, 1998) to identify genes that are differentially expressed in selectively vulnerable cell populations in the inferior temproal cortex with confirmed neurodegeneration and in the largely unaffected frontal or sensory-motor cortex of the same subject in three brains with a histopathological diagnosis of Alzheimer's disease and post mortem time intervlas of less than four hours. By using forty different primer combinations, twenty-eight of thirty-six differentially expressed cDNAs were cloned and sequenced. These cDNAs were further analyzed by reverse Northern blotting (Poirier G.M.-C. et al., Nucleic Acid Res., 25, 913, 1997; Van Gelder R. N. et al., Proc. Natl. Acad. Sci. USA, 87, 1663, 1990) to confirm differential expression between the two AD brain regions. Expression of one of these cDNAs was markedly lower in the inferior temporal lobe than in the sensory-motor cortex. Therefore, the potential importance of this transcript for the selective vulnerability in AD brain has been investigated. The cDNA sequence consisted of 4248 nucleotides and encoded an open reading frame of 516 amino acid residues. Due to a cytidine insertion at nucleotide position 1167, this sequence differed from the much shorter coding region of its homolog KIAA0018 deposited in GenBank (Nomura et al., D N A Res. 1, 27, 1994; GenBank database accession HUMRSC390D13643,1, 1992; DIMH Human Q15392, 1998). The new gene has been designated *SELADIN-1*. The homology domain to oxido-reductases are highlighted in red; the homologies to "diminuto like proteins" of other species are underlined. The first 21 amino acid residues represent a putative signal peptide. One possible caspase recognition motif is highlighted in yellow. This putative caspase recognition motif "LEVD" is present within the SELADIN-1 amino acid sequence at position 121 - 125. *In vitro* cleavage of SELADIN-1 by caspase 3 or 6 generated four different SELADIN-1 fragments of approximately 50, 40, 30 and 20 kDa, respectively. Secondary structure predictions revealed at least four possible transmembrane domains.

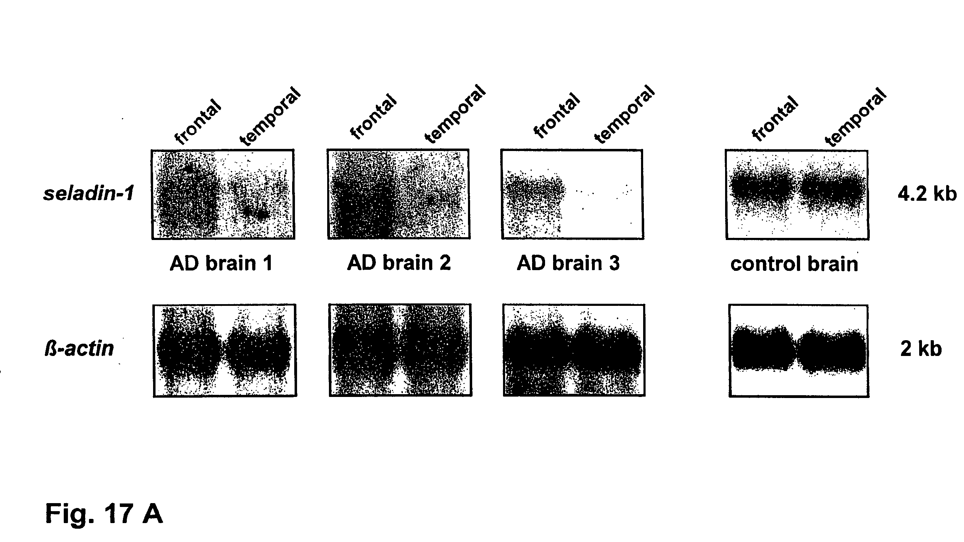

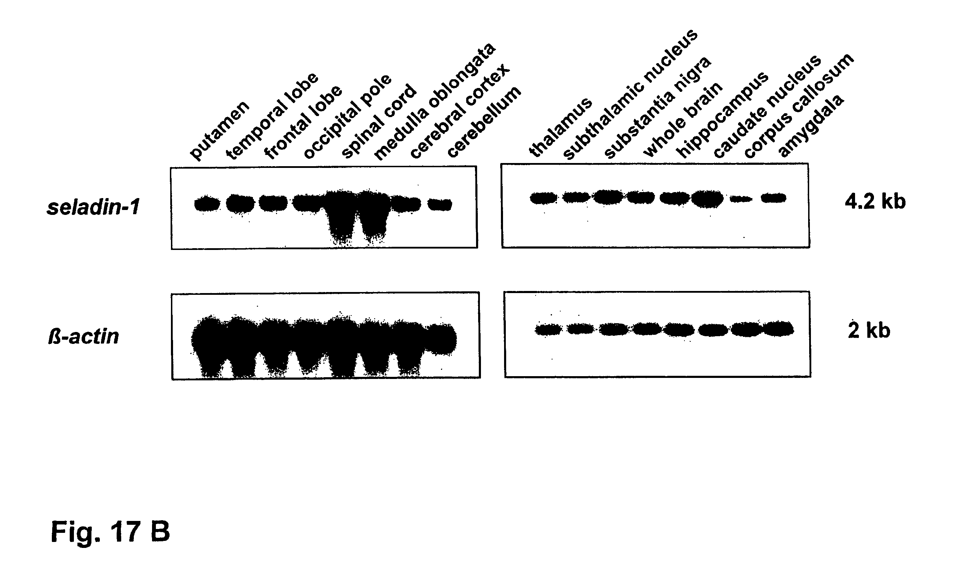

Figure 17 shows Northern blots of Alzheimer's disease (AD) brain and normal control brain. In AD brains, the expression of *SELADIN-1* was substantially lower in the inferior temporal lobe compared to the frontal cortex. In contrast, there was no difference in expression between these two regions in normal control brains (Fig. 17 A, B). Thus, the differential expression of *SELADIN-1* between temporal and frontal cortex within individual AD brains initially observed by both differential display and reverse Northerns, was independently confirmed in three other patients. *SELADIN-1* is strongly expressed throughout the normal human brain with highest expression in the cortices, in the medulla oblongata and the spinal cord as well as in substantia nigra and the hippocampus (Fig. 17B). **A** 10 µg of total RNA per lane, extracted with Trizol Reagent (Gibco) from the frontal cortex or the inferior temporal cortex of three different AD brains were separated on a 0.8 % formaldehyde-agarose gel and blotted on a Hybond-N+-Nylon Membrane (Amersham). Brain 1: post mortem time interval 3:30 hours, male, 72 years. Brain 2: post mortem time interval 1:30 hours, male, 62 years. Brain 3: post mortem time interval 4 hours, female, 63 years. Control brain: normal brain, post mortem time interval 1:10 hours, female, 80 years. The blots were hybridized with a ³²P-labeled c D N A probe of *Seladin-1* from nucleotide 1 - 3505 and with a ³²P-labeled c D N A control probe of human β-actin as provided by Clontech for the human brain multiple tissue northern blot II and III. **B** Human brain multiple tissue Northern blot II (Clontech 7755-1) and III (Clontech 7750-1) containing 2 µg of polyA+ RNA per lane from 16 different human brain regions. Blots were hybridized with the same probes as described in A.

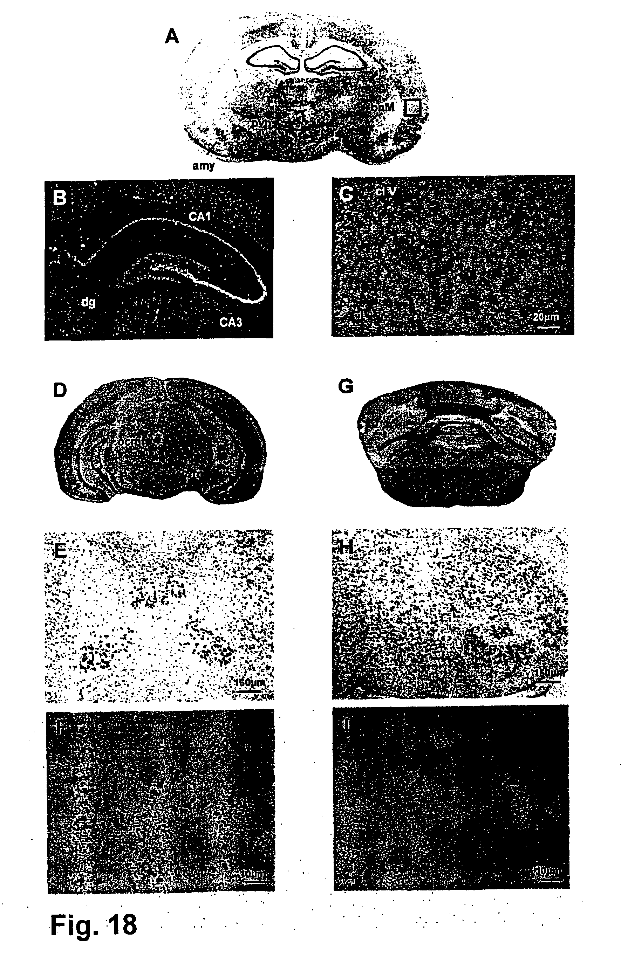

Figure 18 shows the expression of *Seladin-1* in rat brain. *In situ* hybridization on paraformaldehyde fixed cryostat sections was performed as described by Hartman et al. (Developmental Neuroscience 17, 246, 1995). A 650 bp and a 900 bp fragment of the open reading frame of *Seladin-1* were PCR amplified using the following primer pairs: 1s (76-99) 5' GCG CTT ACC GCG CGG CGC CGC ACC 3' (SEQ ID NO. 3) 1 as (749-726) 5' GAC CAG GGT ACG GCA TAG AAC AGG 3' (SEQ ID NO. 4) 3s (803-826) 5' AGA AGT ACG TCA AGC TGC GTT TCG 3' (SEQ ID NO. 5) and 3as (1749-1726) 5' TTC TCT TTG AAA GTG TGG ATC TAG 3' (SEQ ID NO. 6). PCR fragments were cloned in pGEM-Teasy vector (Promega), cut with EcoRI and cloned in pBluescript KS+. The orientation of the EcoRI cloned fragments was analyzed by PCR. Using the Ambion Maxiscript kit, ³⁵S-UTP labeled antisense and sense riboprobes were generated on Notl and Clal linearized plasmids with T3 and T7-Polymerase, respectively, according to the manufacturers instructions. Hybridized sections were dipped in NTB-3 photographic emulsion (Kodak), exposed for 5 weeks and counterstained in Mayer's hemalum. **A, D, G** show photomicrographs of the emulsion dipped sections. **pvn** paraventricular nucleus, **bnM** basal nucleus of Meynert, **amy** amygdala, ocmn oculomotor nucleus, **rn** red nucleus, **fn** facial nucleus. **B** is a darkfield illumination blow up of the hippocampal region. **dg** dentate gyrus. **C** is a darkfield illumination blow up of the cortical layer five **cl V. E, H** show brightfield higher magnification photomicrographes of the regions of interest from D and G. **F**, **I** DIC (differential interference contrast) illuminations in higher magnification of E and H to demonstrate single neurons stained with silver grains. In rat brain, expression of *SELADIN-1* was high in the hippocampal region CA3 (Fig. 18 A, B), in the pyramidal neurons of cortical layer five (Fig. 18 A, C), in the amygdala (Fig. 18 A), in the magnocellular neurons of the basal nucleus of Meynert (Fig. 18 A) and in the reticular zone of the substantia nigra (data not shown). In addition, transcripts were also detected in several brain nuclei including the paraventricular nucleus (Fig. 18 A), the oculomotor nucleus (Fig. 18 D, E), the facial nucleus (Fig. 18 G, F) as well as the red nucleus (Fig. 18 D, E).

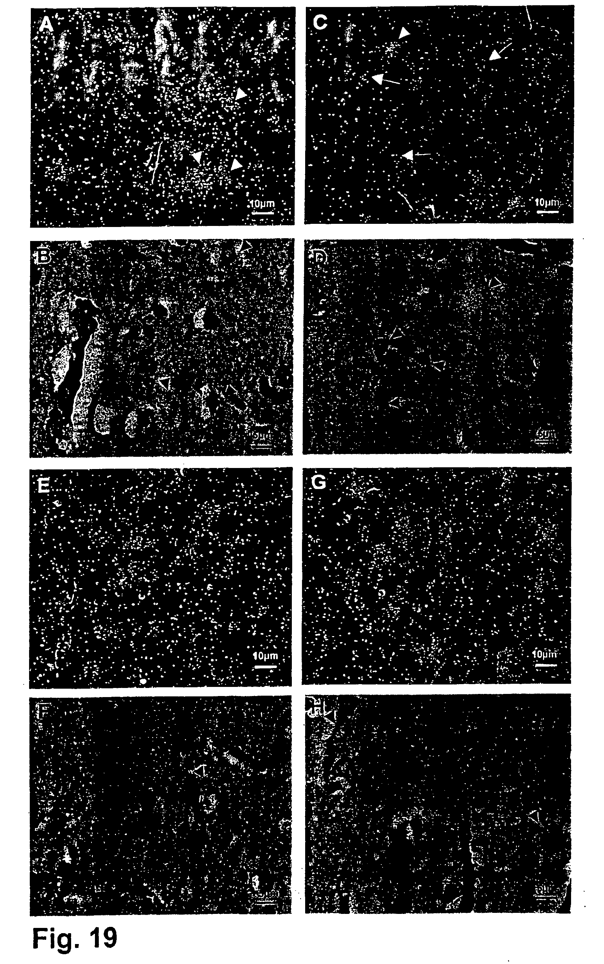

Figure 19 shows *in situ* hybridization of human AD (**A-D**) and normal brain (**E-H**). *In situ* hybridization on embedded sections was performed as described (U. Süsens, Dev. Neurosci. 19, 410, 1997). The ³⁵S-UTP labeled riboprobe was derived from the first 650 nucleotides of the open reading frame of *Seladin-1* cloned in pBluescript KS+ as described in Figure 18. The hybridized slides were dipped in Kodak NTB-2 emulsion, exposed for 4 weeks. After development, sections were stained with Giemsa. **A, C, E** and **G** show darkfield illuminations and **B, D, F, H** the corresponding brightfield photomicrographes. To enhance the visibility of the silver grains in the brightfield picture higher magnification is shown. **A, B** representative hybridization pattern of *Seladin-1* in midfrontal cortex of AD brain. **C, D** representative hybridization pattern of *Seladin-1* in superior temporal cortex of AD brain. **E, F** representative hybridization pattern of *Seladin-1* in midfrontal cortex of normal brain. **G, H** representative hybridization pattern of *Seladin-1* in superior temporal cortex of normal brain. Arrowheads indicate neurons packed with silver grains; arrows indicate the neurons with only few grains (D). *In situ* hybridization of human AD and control brains to study the expression of *SELADIN-1* within single neurons, demonstrated that *SELADIN-1* mRNA was reduced in the remaining neurons of the temporal cortex in comparison to the neurons in the frontal cortex in the AD brains (Fig. 19, A-D, arrows). In contrast, in normal brains, neuronal expression of *SELADIN-1* was identical between the frontal cortex and the temporal cortex (Fig. 19, E-H, arrowheads), confirming the data from differential display and Northern blot analyses. Reduced levels of *SELADIN-1* mRNA in the temporal cortex in comparison to the frontal cortex in the AD brain were not only due to cell loss but were also reduced within the remaining neurons.

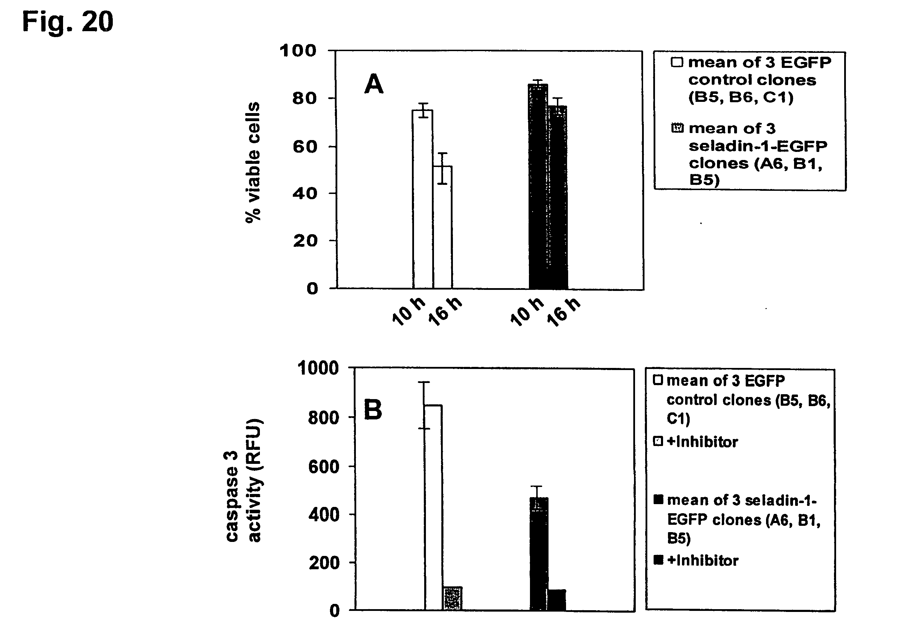

Figure 20. To analyze SELADIN-1 function as a putative oxido-reductase, human H4 neuroglioma cells were stably transfected with Seladin-1 fused at its C-terminus to EGFP (enhanced green fluorescence protein, Clontech). **A** 10 and 16 hours after incubation of three seladin-1-EGFP clones and three EGFP-control clones in OptiMEM1 containing 200 µM H₂O₂, cells remaining attached to the culture dish as well as cells in the supernatant were harvested and stained with 7-Amino-actinomycin D (7-ADD) as a standard flow cytometric viability probe to distinguish viable from non viable cells. Only membranes of dead and damaged cells are permeable to this D N A dye and stain positive. Live/dead counts were done on FACSCalibur (Becton Dickinson) counting 10⁵ cells per clone. Means of 2 experiments in triplicate are shown (± SEM). All SELADIN-1 expressing clones tolerated H₂O₂-induced oxidative stress much better than either non-transfected or EGFP expressing clones. After ten hours treatment with 200 µM H₂O₂ nearly 90 % of the SELADIN-1 expressing cells and 75 - 80 % of the control cells were viable; sixteen hours after incubation with 200 µM H₂O₂, however, 80 % of the SELADIN-1 expressing cells were still alive whereas only 52 % of the control cells were alive at this time point. Untreated control clones revealed a maximum of 5 % dead cells at equivalent time intervals. Increased survival rates in SELADIN-1 expressing cells after prolonged exposure to oxidative stress was confirmed by two independent approaches: First, live/dead counts were done on trypan blue stained cells on cell culture dishes and visualized in phase-contrast microscopy in ten randomly chosen fields. Second, nuclei of cells grown on coverslips and fixed with 4 % paraformaldehyde were stained with Hoechst dye 33342 (Molecular Probes) and visualized by fluorescence microscopy (data not shown). These measures confirmed that expression of SELADIN-1 conferred resistance against induction of cell death.
   **B** To determine an early marker for apoptotic cell death, the activity of caspase 3 in cell lysates of three SELADIN-1-EGFP clones and three EGFP-control clones was measured using the caspase 3 assay kit from Pharmingen. After induction of apoptosis with 200 µM H₂O₂ for 2 or 4 hours, respectively, cells were washed briefly in PBS and lysed in 10 mM Tris-HCl, pH 7.5, 10 mM NaH₂PO₄ pH 7.5, 130 mM NaCl, 1 % Triton-X-100, 10 nM NaPPi (2 million cells/ml). 50 µl of the cell lysates were incubated in 200 µl HEPES buffer for 1 hour at 37 °C with 5 µg of the caspase 3 fluorogenic substrate Ac-DEBD-CHO in a 96 multiwell plate. The AMC liberated from Ac-DEVD after caspase cleavage was measured on a spectrofluorometer (Spectramax Gemini, Molecular Devices) with an excitation wavelength of 380 nm and an emission wavelength spectrum from 420 - 460 nm. Means of caspase 3 activity, measured in RFU (relative fluorescence units) of two experiments in triplicates are shown (± SEM). Two hours after induction of apoptosis with 200 µM H₂O₂, caspase 3 activity was not detectable in either SELADIN-1-EGFP clones or in the EGFP-control clones. After 4 hours, however, the activity of caspase 3 strongly increased and was found to be approximately two-fold higher in three EGFP-control clones as compared to three SELADIN-1-EGFP clones. This increase in caspase 3 activity was blocked in either condition by the caspase inhibitor Ac-DEVD-CHO.

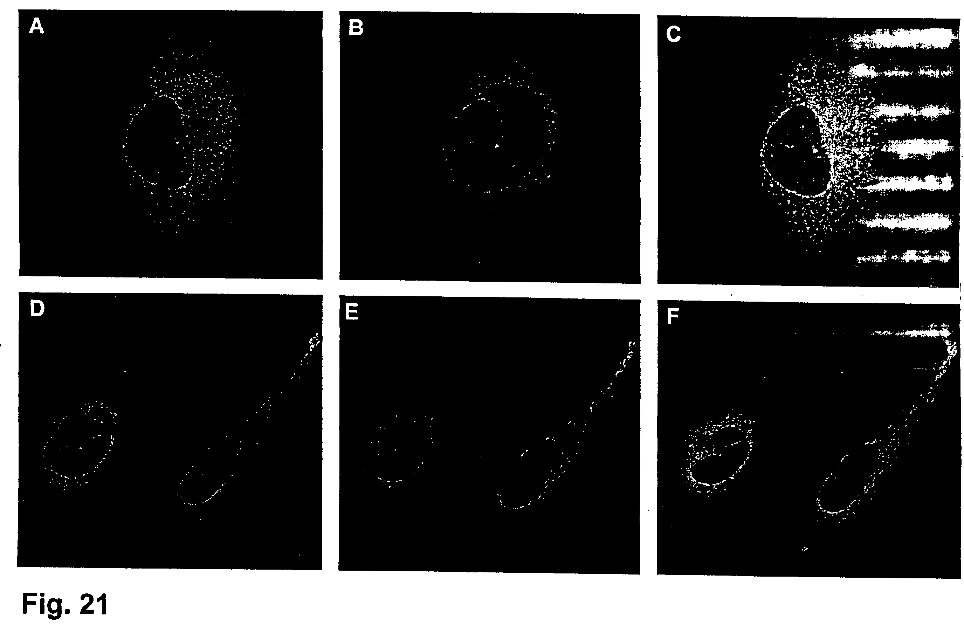

Figure 21 shows the subcellular localization of SELADIN-1. 114 human neuroglioma cells that stable express a fusionprotein of SELADIN-1 with the N-terminus of EGFP (Clontech) were grown on coverslips and fixed in 4 % paraformaldehyde in PBS or treated for 45 minutes with 250 nM of the red fluorescent mitochondrial stain MitoTracker red CM H₂Xros (Molecular Probes) before fixation. After fixation cells that have not been prestained with the MitoTracker were permeabilized in 0.2 % Triton-X 100 in PBS and blocked over night at 4 °C in 5 % low fat milk, 0.1 % Triton-X 100 in PBS. Cells were incubated for 2 hours at room temperature with an monoclonal antibody against the mouse anti-protein disulfide isomerase (antiPDlmAb, StressGen Biotechnologies Corp.), a marker for the endoplasmic reticulum, washed and incubated for another hour with an anti-mouse IgG, CY3 labeled secondary antibody (Amersham). Cells were visualized with confocal laser scanning microscopy. **A, D** Subcellular distribution of the green fluorescent SELADIN-1-EGFP fusionprotein. **B** Staining of the endoplasmatic reticulum with the antiPDlmAb and the red fluorescent CY3 labeled secondary antibody. **C** Overlay from A and B shows the colocalization of SELADIN-1 with the ER-marker, indicated as yellow fluorescence. **E** Staining of the mitochondria with the red fluorescence MitoTracker CM H₂Xros. **F** Overlay of D and E. These colocalization studies with markers and antibodies against several subcellular organelles indicated that SELADIN-1-EGFP mainly localized to the endoplasmatic reticulum and not to the mitochondria, despote the presence of a putative mitochondrial localization signal at the N-terminus of SELADIN-1.

Taken together a novel gene *SELADIN-1* that has homologies to FAD-dependent oxido-reductases has been identified. It has been shown that it was down-regulated in selectively vulnerable regions of AD brain. *In situ* hybridization of AD brain sections demonstrated that the reduced mRNA levels are not only due to neuronal loss in affected areas but also reflects reduced mRNA expression of the remaining neurons. Expression of *SELADIN-1* in H4 cells conferred resistance to apoptosis by oxidative stress, yet after execution of apoptosis SELADIN-1 is cleaved at putative caspase cleavage sites and therefore is presumably inactivated. These results indicate that SELADIN-1 is an integral component of the cellular machinery protecting cells, in particular neurons, from oxidative stress. Once oxidative stress becomes overwhelming, SELADIN-1 becomes a target for caspase action in the course of apoptosis. *SELADIN-1* is a good candidate gene for therapeutical intervention to protect cells against degeneration and cell death. It is in particular, a good candidate gene for therapeutical intervention to protect neurons from Aβ induced cytotoxicity.

### EXAMPLE I

### Post-mortem Alzheimer's disease brain tissues

Brain tissues from Alzheimer's disease patients and control subjects were removed within 6 hours of death, and immediately frozen on dry ice. Parallel sections were fixed in formaldehyde for histopathological confirmation of the diagnosis and for cell counts. Brain areas with massive neuronal cell loss as well as areas with largely preserved neurons were identified for comparisons of gene expression and stored at -80°C until RNA extractions were performed.

### Identification of SELADIN-1 by differential display PCR

Total RNA from post-mortem brain tissues was prepared by using the RNeasy kit (Qiagen). The RNA preparations were treated with DNase I (Boehringer Mannheim) together with RNAsin (Promega) for 30 minutes, followed by phenol extraction, and ethanol precipitation. 0.2 mg of each RNA preparation were transcribed to cDNA by using Expand Reverse Transcriptase (Boehringer Mannheim) with one base ancor primers HT₁₁A, HT₁₁C and HT₁₁G. In the following PCR reaction, the cDNAs were amplified by using HT₁₁A along with the random primers HAP-5 (5'-TGCCGAAGCTTGGAGCTT-3') and HAP3-T (5'TGCCGAAGCTTTGGTCAT-3'). Taq-polymerase (AmpliTaq, Perkin Elmer Corp.), dGTP, dCTP, and dTTP (Amersham Pharmacia Biotech) and (α³⁵S)-dATP (NEN life science products) were used in a PCR protocol according to Zhao et al. The PCR products were separated on 6% polyacrylamide-urea sequencing gels that were dried subsequently on 3 mm filter paper (Whatman), and X-ray films (Dupont) were exposed for 12 hours.

### Cloning and sequencing

Differential bands were excised from the gel, boiled in water for 10 minutes, centrifuged, and cDNAs were precipitated from the supernatant fluids by using ethanol and glycogen/sodiumacetate, followed by dialysis against 10% glycerol for 1 hour through 0.025 mm filters (type VS, Millipore). The dialysates were used as templates for the reamplification reactions that were done under identical conditions as in the differential display PCR, with the exception of the initial cycle for nonspecific annealing. The resulting PCR products were separated by agarose gelelectrophoresis, purified from the gel with the QIAEXII Agarose Gel Extraction Kit (Qiagen), and cloned into the *Hind* III restriction site of pBluescript KS (Stratagene). Cloned cDNA fragments were sequenced with an ABI 377 DNA sequencer (Perkin Elmer Corp.) by using T3 and T7 primers.

### Amplification of a SELADIN-1 cDNA-fragment

*A SELADIN-1* cDNA fragment was amplified by using cDNA transcribed from human brain tissue by using RNA High Fidelity Taq-polymerase (Boehringer Mannheim) and *SELADIN-1*-specific primers for a PCR reaction with 40 cycles of annealing of 70 °C for 1 minute, and polymerization at 72 °C for 3 minutes. The PCR products were separated by agarose gel electrophoresis, purified, and cloned into the *Sma* I restriction site of pBluescript KS (Stratagene). The cloned PCR product was sequenced, and restriction were used as a probe both for screening a human brain cDNA library and for probing Northern blots.

### Northern blotting

Total RNA from post-mortem human brains were prepared by using the Trizol reagent (Gibco BRL, Life Technologies), following the manufacturer's instructions. 5 - 10 mg of RNA were separated in 1 % formaldehyde-containing agarose gels, and the RNA was blotted onto nylon membranes (Hybond-N⁺, Amersham). Membranes were hybridized with (α-³²P)-dCTP (NEN) labeled SELADIN-1-specific cDNA probes that were generated by using the Megaprime DNA labelling kit (Amersham). Membranes were washed under high stringency conditions, and X-ray films were exposed for 1 to 72 hours. To control for equal loading of RNA, the identical membranes were probed with a 700 pb cDNA fragment of human glycerolaldehyd-3-phosphate dehydrogenase (*GAPDH*), or with a b-actin cDNA fragment (Clontech).

### In situ hybridization

Several *SELADIN*-1-specific cDNA probes of 650bp and of 900bp representing the initial two parts of the open reading frame were cloned in pBluescript (Stratagene) and reversely transcribed in the presence of ³⁵S-CTP by using the Ambion transcription kit. In situ hybridization was done with, 14mm sections of adult rat brain cut on a cryomicrotome, mounted on aminoalkylsilane-treated slides and fixed in 4 % paraformaldehyde in PBS for 5 min at room temperature. After washing for 5 min in PBS, sections were acetylated for 10 min, passed through a series of increasing ethanol grades and air dried. Prehybridizations were done in 50 % deionized formamide, 25 mM EDTA, 25 mM Pipes, pH 6.8, 0.75 M NaCl, 0.2 % SDS, 5 x Denhardt's, 10 mM DTT, 250 mg/ml denatured herring sperm DNA and 250 mg/ml yeast tRNA. Hybridization of slides with RNA sense and antisense probes diluted to 2000 - 5000 cpm/ml in the same buffer with additional 10 % dextransulphate was performed at 50 °C for 12 hours. Slides were then washed four times in 4 x SCC for 5 min. each, followed by an incubation for 30 min. at 37 °C with 40 mg/ml RNAseA in 0.5 M NaCl, 10 mM Tris-HCL, pH 7.5, 1 mM EDTA and another 30 min without RNAseA. Then slides were washed twice for 15 min. at 50 °C in 2 x SCC and dried through graded ethanols. Slides were exposed to Kodak Biomax x-ray films for 15 days and subsequently dipped in Kodak NTB-3 nuclear track emulsion and exposed for 6 weeks. After developing in Kodak D19 and fixing in Kodak Unifix, slides were counterstained with Mayer" Hemalaun and coversplipped.

### Recombinant expression of SELADIN-1-EGFP fusion proteins in tissue culture

The complete coding region of *SELADIN-1* was subcloned into the N-terminus of the pEGFP-N1-expression vector (Clontech). Cos-7-cells were transfected with *EGFP* or with *SELADIN-1-EGFP* by using the SuperFect transfection reagent from Qiagen according to the manufacturers instructions. Cells were cultured in 3 cm dishes for two days. Part of the cells were stained for the Golgi-apparatus with 0.25 mM BODIPY TR ceramide (molecular probes) for one hour, the other part was treated with 250 nM of the mitochondrial stain Mito Tracker Red CM-H2Xros (Molecular probes) for 45 min. the subcellular localization of the SELADIN-1-EGFP fusion protein was analyzed by confocal laser scanning microscopy using the appropriate filter sets.

## Claims

1. An isolated nucleic acid encoding a protein molecule shown in SEQ ID NO. 1.

2. An isolated nucleic acid molecule encoding a protein molecule, the function of which is to protect cells against degeneration and/or cell death, wherein the amino acid sequence of the protein molecule comprises the sequence shown in SEQ ID NO.1 or a functional variant thereof.

3. An isolated nucleic acid molecule of claim 1 or 2, wherein the nucleic acid molecule is a D N A molecule.

4. An isolated nucleic acid molecule of claim 3, wherein the nucleic acid molecule is a cD N A molecule.

5. An isolated D N A molecule encoding a protein molecule, the function of which is to protect cells against degeneration and/or cell death, capable of hybridizing with the complement of the cD N A described in SEQ ID NO. 2 under stringent condition.

6. An isolated nucleic acid molecule of claim 2 or 5 encoding a protein molecule, the function of which is to protect cells of the nerve system, muscular system, prostate, stomach, testis, ovary, adrenal glands, mammary glands, liver, spleen, lung, trachea or placenta against degeneration and/or cell death.

7. A vector comprising a nucleic acid molecule according to one of claims 1 to 6.

8. A vector according to claim 7 wherein said vector is a plasmid, a virus or a bacteriophage.

9. A plasmid according to claim 8 wherein said plasmid is adapted for expression in a yeast cell and further comprises the regulatory elements necessary for expression of said nucleic acid molecule.

10. A plasmid according to claim 8 wherein said plasmid is adapted for expression in a bacterial cell and further comprises the regulatory elements necessary for expression of said nucleic acid molecule.

11. A plasmid according to claim 8 wherein said plasmid is adapted for expression in a mammalian cell and further comprises the regulatory elements necessary for expression of said nucleic acid molecule.

12. A cell transformed with a nucleic acid molecule according to one of claims 1 to 6.

13. A cell according to claim 12, wherein said cell is a bacterial cell, a yeast cell, a mammalian cell, or an insect cell.

14. A protein molecule shown in SEQ ID NO.1.

15. A protein molecule, the function of which is to protect cells against degeneration and/or cell death, wherein the amino acid sequence of the protein molecule comprises the sequence shown in SEQ ID NO.1 or a functional variant thereof.

16. A protein molecule of claim 14, the function of which is to protect cells of the nerve system, muscular system, prostate, stomach, testis, ovary, adrenal glands, mammary glands, liver, spleen, against degeneration and/or cell death.

17. An antibody specifically immunoreactive with an immunogen, wherein said immunogen is a protein molecule shown in SEQ ID NO. 1.

18. An antibody specifically immunoreactive with a protein molecule, the function of which is to protect cells against degeneration and/or cell death, wherein the amino acid sequence of the protein molecule comprises the sequence shown in SEQ ID NO. 1 or a functional variant thereof.

19. An in-vitro method of detecting pathological cells in a subject which comprises immunocyto-chemically staining cells with an antibody of claim 17 or 18, wherein a low degree of staining in said cell compared to a cell representing a known health status indicates a pathological change of said cells.

20. A method of claim 19, wherein cells of the nerve system, muscular system, prostate, stomach, testis, ovary, adrenal glands, mammary glands, liver, spleen, lung, trachea or placenta are used.

21. An in-vitro method of diagnosing or prognosing a disease, in a subject, said method comprising:
determining a level, or an activity, or both said level and said activity, of at least one substance which is selected from the group consisting of
(a) a D N A molecule encoding a protein molecule, the function of which is to protect cells against degeneration and/or cell death, wherein the amino acid sequence of the protein molecule comprises the sequence shown in SEQ ID NO.1 or a functional variant thereof,
(b) a transcription product of a D N A molecule encoding a protein molecule, the function of which is to protect cells against degeneration and/or cell death, wherein the amino acid sequence of the protein molecule comprises the sequence shown in SEQ ID NO.1 or a functional variant thereof,
(c) a protein molecule, the function of which is to protect cells against degeneration and/or cell death, wherein the amino acid sequence of the protein molecule comprises the sequence shown in SEQ ID NO.1 or a functional variant thereof,
(d) a D N A molecule encoding a protein molecule, the function of which is to protect cells against degeneration and/or cell death, capable of hybridizing with the complement of the c D N A described in SEQ ID NO. 2 under stringent conditions,
(e) a transcription product of a D N A molecule, encoding a protein molecule, the function of which is to protect cells against degeneration and/or cell death, capable of hybridizing with the complement of the c D N A described in SEQ ID NO. 2 under stringent conditions,
(f) a translation product of a D N A molecule, encoding a protein molecule, the function of which is to protect cells against degeneration and/or cell death capable of hybridizing with the complement of the c D N A described in SEQ ID NO. 2 under stringent conditions,
and comparing said level, or said activity, or both said level and said activity, of at least one of said substances (a) to (f) to a reference value representing a known disease or health status, thereby diagnosing or prognosing a disease in said subject.

22. An in-vitro method of monitoring the progression of a disease, in a subject, said method comprising:
determining a level, or an activity, or both said level and said activity, of at least one substance which is selected from the group consisting of
(a) a D N A molecule encoding a protein molecule, the function of which is to protect cells against degeneration and/or cell death, wherein the amino acid sequence of the protein molecule comprises the sequence shown in SEQ ID NO.1 or a functional variant thereof,
(b) a transcription product of a D N A molecule encoding a protein molecule, the function of which is to protect cells against degeneration and/or cell death, wherein the amino acid sequence of the protein molecule comprises the sequence shown in SEQ ID NO.1 or a functional variant thereof,
(c) a protein molecule, the function of which is to protect cells against degeneration and/or cell death, wherein the amino acid sequence of the protein molecule comprises the sequence shown in SEQ ID NO.1 or a functional variant thereof,
(d) a D N A molecule, encoding a protein molecule, the function of which is to protect cells against degeneration and/or cell death, capable of hybridizing with the complement of the c D N A described in SEQ ID NO. 2 under stringent conditions,
(e) a transcription product of a D N A molecule, encoding a protein molecule, the function of which is to protect cells against degeneration and/or cell death, capable of hybridizing with the complement of the c D N A described in SEQ ID NO. 2 under stringent conditions,
(f) a translation product of a D N A molecule, encoding a protein molecule, the function of which is to protect cells against degeneration and/or cell death, capable of hybridizing with the complement of the c D N A described in SEQ ID NO. 2 under stringent conditions,
and comparing said level, or said activity, or both said level and said activity, of at least one of said substances (a) to (f) to a reference value representing a known disease or health status, thereby monitoring progression of a disease in said subject.

23. Au in-vitro method of evaluating a treatment for a disease, in a subject, said method comprising:
determining a level, or an activity, or both said level and said activity, of at least one substance which is selected from the group consisting of
(a) a D N A molecule encoding a protein molecule, the function of which is to protect cells against degeneration and/or cell death, wherein the amino acid sequence of the protein molecule comprises the sequence shown in SEQ ID NO.1 or a functional variant thereof,
(b) a transcription product of a D N A molecule encoding a protein molecule, the function of which is to protect cells against degeneration and/or cell death, wherein the amino acid sequence of the protein molecule comprises the sequence shown in SEQ ID NO.1 or a functional variant thereof,
(c) a protein molecule, the function of which is to protect cells against degeneration and/or cell death, wherein the amino acid sequence of the protein molecule comprises the sequence shown in SEQ ID NO.1 or a functional variant thereof,
(d) a D N A molecule, encoding a protein molecule, the function of which is to protect cells against degeneration and/or cell death, capable of hybridizing with the complement of the c D N A described in SEQ ID NO. 2 under stringent conditions,
(e) a transcription product of a D N A molecule, encoding a protein molecule, the function of which is to protect cells against degeneration and/or cell death, capable of hybridizing with the complement of the c D N A described in SEQ ID NO. 2 under stringent conditions,
(f) a translation product of a D N A molecule, encoding a protein molecule, the function of which is to protect cells against degeneration and/or cell death, capable of hybridizing with the complement of the c D N A described in SEQ ID NO. 2 under stringent conditions,
and comparing said level, or said activity, or both said level and said activity, of at least one of said substances (a) to (f) to a reference value representing a known disease or health status, thereby evaluating a treatment for a disease in said subject.

24. The method according to one of claims 21 to 23, wherein a decrease of a level or an activity of (i) a transcription product of a D N A molecule encoding a protein molecule, the amino acid sequence of which comprises the sequence shown in SEQ ID NO.1 or a functional variant thereof or (ii) a protein molecule, the amino acid sequence of which comprises the sequence shown in SEQ ID NO.1 or a functional variant thereof, in a sample from said subject relative to a reference value representing a known health status indicates the presence of a disease, in said subject.

25. The methods according to claims 21 to 24 wherein said disease is a neurological disease.

26. The method according to one of claims 21 to 25, wherein said subject suffers from Alzheimer's disease or related neurofibrillary disorders, or neurodegenerative states **characterized by** cell degeneration or cell death, or Parkinson's disease, or Huntington disease, or amyotrophic lateral sclerosis, or Pick's disease.

27. A method of identifying an agent that affects an activity, or level, or both said activity and level, of at least one substance which is selected from the group consisting of
(a) a D N A molecule encoding a protein molecule, wherein the amino acid sequence of the protein molecule comprises the sequence shown in SEQ ID NO.1 or a functional variant thereof,
(b) a transcription product of a D N A molecule encoding a protein molecule, wherein the amino acid sequence of the protein molecule comprises the sequence shown in SEQ ID NO.1 or a functional variant thereof,
(c) a protein molecule, wherein the amino acid sequence of the protein molecule comprises the sequence shown in SEQ ID NO.1 or a functional variant thereof,
(d) a D N A molecule capable of hybridizing with the complement of the c D N A described in SEQ ID NO. 2 under stringent conditions,
(e) a transcription product of a D N A molecule capable of hybridizing with the complement of the c D N A described in SEQ ID NO. 2 under stringent conditions,
(f) a translation product of a D N A molecule capable of hybridizing with the complement of the c D N A described in SEQ ID NO. 2 under stringent conditions,
comprising the steps of:
(i) providing a sample containing at least one substance which is selected from the group consisting of (a) to (f),
(ii) contacting said sample with at least one agent,
(iii) comparing an activity, or level, or both said activity and level, of at least one of said substances before and after contacting.

28. A method of claim 27 wherein the function of said protein molecule or a variant thereof is to protect cells from degeneration and/or cell death.

29. A method of claim 27 or 28 wherein said D N A molecule capable of hybridizing with the complement of the c D N A described in SEQ ID NO. 2 encodes a protein molecule, the function of which is to protect cells against degeneration and/or cell death.

30. A kit for diagnosis, or prognosis of a disease, said kit comprising:
(1) at least one reagent which is selected from the group consisting of reagents that selectively detect
(a) a D N A molecule encoding a protein molecule, wherein the amino acid sequence of the protein molecule comprises the sequence shown in SEQ ID NO.1 or a functional variant thereof,
(b) a transcription product of a D N A molecule encoding a protein molecule, wherein the amino acid sequence of the protein molecule comprises the sequence shown in SEQ ID NO.1 or a functional variant thereof,
(c) a protein molecule, wherein the amino acid sequence of the protein molecule comprises the sequence shown in SEQ ID NO.1 or a functional variant thereof,
(d) a D N A molecule capable of hybridizing with the complement of the c D N A described in SEQ ID NO. 2 under stringent conditions,
(e) a transcription product of a D N A molecule, wherein said D N A molecule is capable of hybridizing with the complement of the c D N A described in SEQ ID NO. 2 under stringent conditions,
(f) a translation product of a D N A molecule, wherein said D N A molecule is capable of hybridizing with the complement of the c D N A described in SEQ ID NO. 2 under stringent conditions,
(2) instructions for diagnosing, or prognosing said disease by
(i) detecting a level, or an activity, or both said level and said activity, of at least one substance which is selected from the group consisting of (a) to (f) in a sample from said subject; and
(ii) diagnosing, or prognosing said disease, wherein a varied level, or activity, or both said level and said activity, of at least one substance which is selected from the group consisting of (a) to (f) compared to a reference value representing a known health status; or a level, or activity, or both said level and said activity, of at least one substance which is selected from the group consisting of (a) to (f) similar or equal to a reference value representing a known disease status indicates diagnosis, or prognosis of said disease.

## Patentansprüche

1. Isolierte Nucleinsäure, die ein Proteinmolekül codiert, das in SEQ ID Nr. 1 gezeigt ist.

2. Isoliertes Nucleinsäuremolekül, das ein Proteinmolekül codiert, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst.

3. Isoliertes Nucleinsäuremolekül gemäß Anspruch 1 oder 2, wobei das Nucleinsäuremolekül ein DNA-Molekül ist.

4. Isoliertes Nucleinsäuremolekül gemäß Anspruch 3, wobei das Nucleinsäuremolekül ein cDNA-Molekül ist.

5. Isoliertes DNA-Molekül, das ein Proteinmolekül, dessen Funktion darin besteht, Zellen vor Degeneration und/oder Zelltod zu schützen, codiert und das unter stringenten Bedingungen mit dem Komplement der in SEQ ID Nr. 2 beschriebenen cDNA hybridisieren kann.

6. Isoliertes Nucleinsäuremolekül gemäß Anspruch 2 oder 5, das ein Proteinmolekül codiert, dessen Funktion darin besteht, Zellen des Nervensystems, der Muskulatur, der Prostata, des Magens, der Hoden, der Eierstöcke, der Nebennieren, der Brustdrüsen, der Leber, der Milz, der Lunge, der Luftröhre oder der Plazenta vor Degeneration und/oder Zelltod zu schützen.

7. Vektor, der ein Nucleinsäuremolekül gemäß einem der Ansprüche 1 bis 6 umfasst.

8. Vektor gemäß Anspruch 7, wobei der Vektor ein Plasmid, ein Virus oder ein Bakteriophage ist.

9. Plasmid gemäß Anspruch 8, wobei das Plasmid für die Expression in einer Hefezelle geeignet ist und weiterhin die für eine Expression des Nucleinsäuremoleküls notwendigen regulatorischen Elemente umfasst.

10. Plasmid gemäß Anspruch 8, wobei das Plasmid für die Expression in einer Bakterienzelle geeignet ist und weiterhin die für eine Expression des Nucleinsäuremoleküls notwendigen regulatorischen Elemente umfasst.

11. Plasmid gemäß Anspruch 8, wobei das Plasmid für die Expression in einer Säugerzelle geeignet ist und weiterhin die für eine Expression des Nucleinsäuremoleküls notwendigen regulatorischen Elemente umfasst.

12. Zelle, die mit einem Nucleinsäuremolekül gemäß einem der Ansprüche 1 bis 6 transformiert ist.

13. Zelle gemäß Anspruch 12, wobei die Zelle eine Bakterienzelle, eine Hefezelle, eine Säugerzelle oder eine Insektenzelle ist.

14. Proteinmolekül, das in SEQ ID Nr. 1 gezeigt ist.

15. Proteinmolekül, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst.

16. Proteinmolekül gemäß Anspruch 14, dessen Funktion darin besteht, Zellen des Nervensystems, der Muskulatur, der Prostata, des Magens, der Hoden, der Eierstöcke, der Nebennieren, der Brustdrüsen, der Leber und der Milz vor Degeneration und/oder Zelltod zu schützen.

17. Antikörper, der gegenüber einem Immunogen spezifisch immunreaktiv ist, wobei das Immunogen ein in SEQ ID Nr. 1 gezeigtes Proteinmolekül ist.

18. Antikörper, der gegenüber einem Proteinmolekül spezifisch immunreaktiv ist, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst.

19. In-vitro-Verfahren zum Nachweisen von pathologischen Zellen bei einem Patienten, das das immunocytochemische Anfärben von Zellen mit einem Antikörper gemäß Anspruch 17 oder 18 umfasst, wobei ein geringes Ausmaß der Anfärbung bei der Zelle im Vergleich zu einer Zelle, die einen bekannten Gesundheitszustand darstellt, auf eine pathologische Veränderung der Zellen hinweist.

20. Verfahren gemäß Anspruch 19, wobei Zellen des Nervensystems, der Muskulatur, der Prostata, des Magens, der Hoden, der Eierstöcke, der Nebennieren, der Brustdrüsen, der Leber, der Milz, der Lunge, der Luftröhre oder der Plazenta verwendet werden.

21. In-vitro-Verfahren zum Diagnostizieren oder Prognostizieren einer Krankheit bei einem Patienten, wobei das Verfahren Folgendes umfasst:
Bestimmen einer Konzentration oder einer Aktivität oder sowohl einer Konzentration als auch einer Aktivität wenigstens einer Substanz, die aus der Gruppe ausgewählt ist, die aus den folgenden besteht:
(a) einem DNA-Molekül, das ein Proteinmolekül codiert, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst;
(b) einem Transcriptionsprodukt eines DNA-Moleküls, das ein Proteinmolekül codiert, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst;
(c) einem Proteinmolekül, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst;
(d) einem DNA-Molekül, das ein Proteinmolekül, dessen Funktion darin besteht, Zellen vor Degeneration und/oder Zelltod zu schützen, codiert und das unter stringenten Bedingungen mit dem Komplement der in SEQ ID Nr. 2 beschriebenen cDNA hybridisieren kann;
(e) einem Transcriptionsprodukt eines DNA-Moleküls, das ein Proteinmolekül, dessen Funktion darin besteht, Zellen vor Degeneration und/oder Zelltod zu schützen, codiert und das unter stringenten Bedingungen mit dem Komplement der in SEQ ID Nr. 2 beschriebenen cDNA hybridisieren kann;
(f) einem Translationsprodukt eines DNA-Moleküls, das ein Proteinmolekül, dessen Funktion darin besteht, Zellen vor Degeneration und/oder Zelltod zu schützen, codiert und das unter stringenten Bedingungen mit dem Komplement der in SEQ ID Nr. 2 beschriebenen cDNA hybridisieren kann; und
Vergleichen der Konzentration oder der Aktivität oder sowohl der Konzentration als auch der Aktivität von wenigstens einer der Substanzen (a) bis (f) mit einem Referenzwert, der einen bekannten Krankheits- oder Gesundheitszustand darstellt, wodurch die Krankheit bei dem Patienten diagnostiziert oder prognostiziert wird.

22. In-vitro-Verfahren zur Überwachung des Fortschritts einer Krankheit bei einem Patienten, wobei das Verfahren Folgendes umfasst:
Bestimmen einer Konzentration oder einer Aktivität oder sowohl einer Konzentration als auch einer Aktivität wenigstens einer Substanz, die aus der Gruppe ausgewählt ist, die aus den folgenden besteht:
(a) einem DNA-Molekül, das ein Proteinmolekül codiert, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst;
(b) einem Transcriptionsprodukt eines DNA-Moleküls, das ein Proteinmolekül codiert, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst;
(c) einem Proteinmolekül, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst;
(d) einem DNA-Molekül, das ein Proteinmolekül, dessen Funktion darin besteht, Zellen vor Degeneration und/oder Zelltod zu schützen, codiert und das unter stringenten Bedingungen mit dem Komplement der in SEQ ID Nr. 2 beschriebenen cDNA hybridisieren kann;
(e) einem Transcriptionsprodukt eines DNA-Moleküls, das ein Proteinmolekül, dessen Funktion darin besteht, Zellen vor Degeneration und/oder Zelltod zu schützen, codiert und das unter stringenten Bedingungen mit dem Komplement der in SEQ ID Nr. 2 beschriebenen cDNA hybridisieren kann;
(f) einem Translationsprodukt eines DNA-Moleküls, das ein Proteinmolekül, dessen Funktion darin besteht, Zellen vor Degeneration und/oder Zelltod zu schützen, codiert und das unter stringenten Bedingungen mit dem Komplement der in SEQ ID Nr. 2 beschriebenen cDNA hybridisieren kann; und
Vergleichen der Konzentration oder der Aktivität oder sowohl der Konzentration als auch der Aktivität von wenigstens einer der Substanzen (a) bis (f) mit einem Referenzwert, der einen bekannten Krankheits- oder Gesundheitszustand darstellt, wodurch der Fortschritt der Krankheit bei dem Patienten überwacht wird.

23. In-vitro-Verfahren zur Bewertung einer Behandlung einer Krankheit bei einem Patienten, wobei das Verfahren Folgendes umfasst:
Bestimmen einer Konzentration oder einer Aktivität oder sowohl einer Konzentration als auch einer Aktivität wenigstens einer Substanz, die aus der Gruppe ausgewählt ist, die aus den folgenden besteht:
(a) einem DNA-Molekül, das ein Proteinmolekül codiert, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst;
(b) einem Transcriptionsprodukt eines DNA-Moleküls, das ein Proteinmolekül codiert, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst;
(c) einem Proteinmolekül, dessen Funktion darin besteht, Zellen vor Degeneration und/oder Zelltod zu schützen, wobei die Aminosäuresequenz des Proteinmoleküls die in SEQ ID Nr. 1 gezeigte Sequenz oder eine funktionelle Variante davon umfasst;
(d) einem DNA-Molekül, das ein Proteinmolekül, dessen Funktion darin besteht, Zellen vor Degeneration und/oder Zelltod zu schützen, codiert und das unter stringenten Bedingungen mit dem Komplement der in SEQ ID Nr. 2 beschriebenen cDNA hybridisieren kann;
(e) einem Transcriptionsprodukt eines DNA-Moleküls, das ein Proteinmolekül, dessen Funktion darin besteht, Zellen vor Degeneration und/oder Zelltod zu schützen, codiert und das unter stringenten Bedingungen mit dem Komplement der in SEQ ID Nr. 2 beschriebenen cDNA hybridisieren kann;
(f) einem Translationsprodukt eines DNA-Moleküls, das ein Proteinmolekül, dessen Funktion darin besteht, Zellen vor Degeneration und/oder Zelltod zu schützen, codiert und das unter stringenten Bedingungen mit dem Komplement der in SEQ ID Nr. 2 beschriebenen cDNA hybridisieren kann; und
Vergleichen der Konzentration oder der Aktivität oder sowohl der Konzentration als auch der Aktivität von wenigstens einer der Substanzen (a) bis (f) mit einem Referenzwert, der einen bekannten Krankheits- oder Gesundheitszustand darstellt, wodurch die Behandlung der Krankheit bei dem Patienten bewertet wird.

24. Verfahren gemäß einem der Ansprüche 21 bis 23, wobei eine Abnahme der Konzentration oder der Aktivität von (i) einem Transcriptionsprodukt eines DNA-Moleküls, das ein Proteinmolekül codiert, dessen Aminosäuresequenz die in SEQ ID Nr. 1 gezeigte Sequenz oder eine funktionelle Variante davon umfasst, oder (ii) einem Proteinmolekül, dessen Aminosäuresequenz die in SEQ ID Nr. 1 gezeigte Sequenz oder eine funktionelle Variante davon umfasst, in einer Probe von dem Patienten relativ zu einem Referenzwert, der einen bekannten Gesundheitszustand darstellt, das Vorhandensein einer Krankheit bei dem Patienten anzeigt.

25. Verfahren gemäß den Ansprüchen 21 bis 24, wobei die Krankheit eine neurologische Krankheit ist.

26. Verfahren gemäß einem der Ansprüche 21 bis 25, wobei der Patient an der Alzheimer-Krankheit oder verwandten neurofibrillären Störungen oder an neurodegenerativen Zuständen, die durch Zelldegeneration oder Zelltod gekennzeichnet sind, oder an der Parkinson-Krankheit oder Chorea Huntington oder amyotropher lateraler Sklerose oder der Niemann-Pick-Krankheit leidet.

27. Verfahren zum Identifizieren eines Mittels, das eine Aktivität oder eine Konzentration oder sowohl eine Aktivität als auch eine Konzentration wenigstens einer Substanz beeinflusst, die aus der Gruppe ausgewählt ist, die aus den folgenden besteht:
(a) einem DNA-Molekül, das ein Proteinmolekül codiert, wobei die Aminosäuresequenz des Proteinmoleküls die in SEQ ID Nr. 1 gezeigte Sequenz oder eine funktionelle Variante davon umfasst;
(b) einem Transcriptionsprodukt eines DNA-Moleküls, das ein Proteinmolekül codiert, wobei die Aminosäuresequenz des Proteinmoleküls die in SEQ ID Nr. 1 gezeigte Sequenz oder eine funktionelle Variante davon umfasst;
(c) einem Proteinmolekül, wobei die Aminosäuresequenz des Proteinmoleküls die in SEQ ID Nr. 1 gezeigte Sequenz oder eine funktionelle Variante davon umfasst;
(d) einem DNA-Molekül, das unter stringenten Bedingungen mit dem Komplement der in SEQ ID Nr. 2 beschriebenen cDNA hybridisieren kann;
(e) einem Transcriptionsprodukt eines DNA-Moleküls, das unter stringenten Bedingungen mit dem Komplement der in SEQ ID Nr. 2 beschriebenen cDNA hybridisieren kann;
(f) einem Translationsprodukt eines DNA-Moleküls, das unter stringenten Bedingungen mit dem Komplement der in SEQ ID Nr. 2 beschriebenen cDNA hybridisieren kann;
wobei das Verfahren die folgenden Schritte umfasst:
(i) Bereitstellen einer Probe, die wenigstens eine Substanz enthält, die aus der Gruppe ausgewählt ist, die aus (a) bis (f) besteht;
(ii) In-Kontakt-Bringen der Probe mit wenigstens einem Reagens;
(iii) Vergleichen der Aktivität oder der Konzentration oder sowohl der Aktivität als auch der Konzentration wenigstens einer der Substanzen vor und nach dem In-Kontakt-Bringen.

28. Verfahren gemäß Anspruch 27, wobei die Funktion des Proteinmoleküls oder seiner Variante darin besteht, Zellen vor Degeneration und/oder Zelltod zu schützen.

29. Verfahren gemäß Anspruch 27 oder 28, wobei das DNA-Molekül, das mit dem Komplement der in SEQ ID Nr. 2 beschriebenen cDNA hybridisieren kann, ein Proteinmolekül codiert, dessen Funktion darin besteht, Zellen vor Degeneration und/oder Zelltod zu schützen.

30. Kit für die Diagnose oder Prognose einer Krankheit, wobei der Kit Folgendes umfasst:
(1) wenigstens ein Reagens, das aus der Gruppe ausgewählt ist, die aus Reagentien besteht, die selektiv Folgendes nachweisen:
(a) ein DNA-Molekül, das ein Proteinmolekül codiert, wobei die Aminosäuresequenz des Proteinmoleküls die in SEQ ID Nr. 1 gezeigte Sequenz oder eine funktionelle Variante davon umfasst;
(b) ein Transcriptionsprodukt eines DNA-Moleküls, das ein Proteinmolekül codiert, wobei die Aminosäuresequenz des Proteinmoleküls die in SEQ ID Nr. 1 gezeigte Sequenz oder eine funktionelle Variante davon umfasst;
(c) ein Proteinmolekül, wobei die Aminosäuresequenz des Proteinmoleküls die in SEQ ID Nr. 1 gezeigte Sequenz oder eine funktionelle Variante davon umfasst;
(d) ein DNA-Molekül, das unter stringenten Bedingungen mit dem Komplement der in SEQ ID Nr. 2 beschriebenen cDNA hybridisieren kann;
(e) ein Transcriptionsprodukt eines DNA-Moleküls, das unter stringenten Bedingungen mit dem Komplement der in SEQ ID Nr. 2 beschriebenen cDNA hybridisieren kann;
(f) ein Translationsprodukt eines DNA-Moleküls, das unter stringenten Bedingungen mit dem Komplement der in SEQ ID Nr. 2 beschriebenen cDNA hybridisieren kann;
(2) Anweisungen zum Diagnostizieren oder Prognostizieren der Krankheit durch:
(i) Bestimmen einer Konzentration oder einer Aktivität oder sowohl einer Konzentration als auch einer Aktivität wenigstens einer Substanz, die aus der Gruppe ausgewählt ist, die aus (a) bis (f) besteht, in einer Probe von dem Patienten; und
(ii) Diagnostizieren oder Prognostizieren der Krankheit, wobei eine veränderte Konzentration oder eine veränderte Aktivität oder sowohl eine veränderte Konzentration als auch eine veränderte Aktivität wenigstens einer Substanz, die aus der Gruppe ausgewählt ist, die aus (a) bis (f) besteht, im Vergleich zu einem Referenzwert, der einen bekannten Gesundheitszustand darstellt, oder eine Konzentration oder Aktivität oder sowohl eine Konzentration als auch eine Aktivität wenigstens einer Substanz, die aus der Gruppe ausgewählt ist, die aus (a) bis (f) besteht, welche ähnlich oder gleich einem Referenzwert ist, der einen bekannten Krankheitszustand darstellt, auf eine Diagnose oder Prognose der Krankheit hinweist.

## Revendications

1. Un acide nucléique isolé codant pour une molécule de protéine représentée dans SEQ ID N° 1.

2. Une molécule d'acide nucléique isolé codant pour une molécule de protéine dont la fonction est de protéger des cellules contre la dégénérescence et/ou la mort cellulaire, dans laquelle la séquence d'acides aminés de la molécule de protéine comprend la séquence représentée dans SEQ ID N° 1 ou un variant fonctionnel de celle-ci.

3. Une molécule d'acide nucléique isolé de la revendication 1 ou 2, dans laquelle la molécule d'acide nucléique est une molécule d'ADN.

4. Une molécule d'acide nucléique isolé de la revendication 3, dans laquelle la molécule d'acide nucléique est une molécule d'ADNc.

5. Une molécule d'ADN isolé codant pour une molécule de protéine dont la fonction est de protéger des cellules contre la dégénérescence et/ou la mort cellulaire, capable de s'hybrider dans des conditions stringentes avec le complément de l'ADNc décrit dans SEQ ID N° 2.

6. Une molécule d'acide nucléique isolé de la revendication 2 ou 5, codant pour une molécule de protéine dont la fonction est de protéger des cellules du système nerveux, du système musculaire, de la prostate, de l'estomac, du testicule, de l'ovaire, des glandes surrénales, des glandes mammaires, du foie, de la rate, du poumon, de la trachée ou du placenta contre la dégénérescence et/ou la mort cellulaire.

7. Un vecteur comprenant une molécule d'acide nucléique selon l'une des revendications 1 à 6.

8. Un vecteur selon la revendication 7, dans lequel ledit vecteur est un plasmide, un virus ou un bactériophage.

9. Un plasmide selon la revendication 8, dans lequel ledit plasmide est fait pour l'expression dans une cellule de levure et comprend de plus les éléments régulateurs nécessaires à l'expression de ladite molécule d'acide nucléique.

10. Un plasmide selon la revendication 8, dans lequel ledit plasmide est fait pour l'expression dans une cellule bactérienne et comprend de plus les éléments régulateurs nécessaires à l'expression de ladite molécule d'acide nucléique.

11. Un plasmide selon la revendication 8, dans lequel ledit plasmide est fait pour l'expression dans une cellule de mammifère et comprend de plus les éléments régulateurs nécessaires à l'expression de ladite molécule d'acide nucléique.

12. Une cellule transformée avec une molécule d'acide nucléique selon l'une des revendications 1 à 6.

13. Une cellule selon la revendication 12, dans laquelle ladite cellule est une cellule bactérienne, une cellule de levure, une cellule de mammifère ou une cellule d'insecte.

14. Une molécule de protéine représentée dans SEQ ID N° 1.

15. Une molécule de protéine dont la fonction est de protéger des cellules contre la dégénérescence et/ou la mort cellulaire, dans laquelle la séquence d'acides aminés de la molécule de protéine comprend la séquence représentée dans SEQ ID N° 1 ou un variant fonctionnel de celle-ci.

16. Une molécule de protéine de la revendication 14, dont la fonction est de protéger des cellules du système nerveux, du système musculaire, de la prostate, de l'estomac, du testicule, de l'ovaire, des glandes surrénales, des glandes mammaires, du foie, de la rate, contre la dégénérescence et/ou la mort cellulaire.

17. Un anticorps spécifiquement immunoréactif avec un immunogène, dans lequel ledit immunogène est une molécule de protéine représentée dans SEQ ID N° 1.

18. Un anticorps spécifiquement immunoréactif avec une molécule de protéine dont la fonction est de protéger des cellules contre la dégénérescence et/ou la mort cellulaire, dans lequel la séquence d'acides aminés de la molécule de protéine comprend la séquence représentée dans SEQ ID N° 1 ou un variant fonctionnel de celle-ci.

19. Une méthode de détection *in vitro* de cellules pathologiques chez un sujet, qui comprend la coloration immunocytochimique de cellules avec un anticorps de la revendication 17 ou 18, dans laquelle un faible degré de coloration dans ladite cellule, comparée à une cellule représentant un état de santé connu, indique un changement pathologique desdites cellules.

20. Une méthode selon la revendication 19, dans laquelle on utilise des cellules du système nerveux, du système musculaire, de la prostate, de l'estomac, du testicule, de l'ovaire, des glandes surrénales, des glandes mammaires, du foie, de la rate, du poumon, de la trachée ou du placenta.

21. Une méthode de diagnostic ou de pronostic *in vitro* d'une maladie chez un sujet, ladite méthode comprenant :
la détermination d'un taux, ou d'une activité, ou à la fois dudit taux et de ladite activité, d'au moins une substance qui est choisie dans le groupe formé par
(a) une molécule d'ADN codant pour une molécule de protéine dont la fonction est de protéger des cellules contre la dégénérescence et/ou la mort cellulaire, dans laquelle la séquence d'acides aminés de la molécule de protéine comprend la séquence représentée dans SEQ ID N° 1 ou un variant fonctionnel de celle-ci,
(b) un produit de transcription d'une molécule d'ADN codant pour une molécule de protéine dont la fonction est de protéger des cellules contre la dégénérescence et/ou la mort cellulaire, dans lequel la séquence d'acides aminés de la molécule de protéine comprend la séquence représentée dans SEQ ID N° 1 ou un variant fonctionnel de celle-ci,
(c) une molécule de protéine dont la fonction est de protéger des cellules contre la dégénérescence et/ou la mort cellulaire, dans laquelle la séquence d'acides aminés de la molécule de protéine comprend la séquence représentée dans SEQ ID N° 1 ou un variant fonctionnel de celle-ci,
(d) une molécule d'ADN codant pour une molécule de protéine dont la fonction est de protéger des cellules contre la dégénérescence et/ou la mort cellulaire, capable de s'hybrider dans des conditions stringentes avec le complément de l'ADNc décrit dans SEQ ID N° 2,
(e) un produit de transcription d'une molécule d'ADN, codant pour une molécule de protéine dont la fonction est de protéger des cellules contre la dégénérescence et/ou la mort cellulaire, capable de s'hybrider dans des conditions stringentes avec le complément de l'ADNc décrit dans SEQ ID N° 2,
(f) un produit de traduction d'une molécule d'ADN, codant pour une molécule de protéine dont la fonction est de protéger des cellules contre la dégénérescence et/ou la mort cellulaire, capable de s'hybrider dans des conditions stringentes avec le complément de l'ADNc décrit dans SEQ ID N° 2,
et la comparaison dudit taux ou de ladite activité, ou à la fois dudit taux et de ladite activité, d'au moins l'une desdites substances (a) à (f) à une valeur de référence représentant un état de maladie ou de santé connu, pour établir ainsi un diagnostic ou un pronostic d'une maladie chez ledit sujet.

22. Méthode de contrôle *in vitro* de l'évolution d'une maladie chez un sujet, ladite méthode comprenant :
la détermination d'un taux, ou d'une activité, ou à la fois dudit taux et de ladite activité, d'au moins une substance qui est choisie dans le groupe formé par
(a) une molécule d'ADN codant pour une molécule de protéine dont la fonction est de protéger des cellules contre la dégénérescence et/ou la mort cellulaire, dans laquelle la séquence d'acides aminés de la molécule de protéine comprend la séquence représentée dans SEQ ID N° 1 ou un variant fonctionnel de celle-ci,
(b) un produit de transcription d'une molécule d'ADN codant pour une molécule de protéine dont la fonction est de protéger des cellules contre la dégénérescence et/ou la mort cellulaire, dans lequel la séquence d'acides aminés de la molécule de protéine comprend la séquence représentée dans SEQ ID N° 1 ou un variant fonctionnel de celle-ci,
(c) une molécule de protéine dont la fonction est de protéger des cellules contre la dégénérescence et/ou la mort cellulaire, dans laquelle la séquence d'acides aminés de la molécule de protéine comprend la séquence représentée dans SEQ ID N° 1 ou un variant fonctionnel de celle-ci,
(d) une molécule d'ADN codant pour une molécule de protéine dont la fonction est de protéger des cellules contre la dégénérescence et/ou la mort cellulaire, capable de s'hybrider dans des conditions stringentes avec le complément de l'ADNc décrit dans SEQ ID N° 2,
(e) un produit de transcription d'une molécule d'ADN, codant pour une molécule de protéine dont la fonction est de protéger des cellules contre la dégénérescence et/ou la mort cellulaire, capable de s'hybrider dans des conditions stringentes avec le complément de fADNc décrit dans SEQ ID N° 2,
(f) un produit de traduction d'une molécule d'ADN, codant pour une molécule de protéine dont la fonction est de protéger des cellules contre la dégénérescence et/ou la mort cellulaire, capable de s'hybrider dans des conditions stringentes avec le complément de l'ADNc décrit dans SEQ ID N° 2,
et la comparaison dudit taux ou de ladite activité, ou à la fois dudit taux et de ladite activité, d'au moins l'une desdites substances (a) à (f) à une valeur de référence représentant un état de maladie ou de santé connu, pour effectuer ainsi le contrôle de la progression d'une maladie chez ledit sujet.

23. Méthode *in vitro* d'évaluation d'un traitement d'une maladie chez un sujet, ladite méthode comprenant :
la détermination d'un taux, ou d'une activité, ou à la fois dudit taux et de ladite activité, d'au moins une substance qui est choisie dans le groupe formé par
(a) une molécule d'ADN codant pour une molécule de protéine dont la fonction est de protéger des cellules contre la dégénérescence et/ou la mort cellulaire, dans laquelle la séquence d'acides aminés de la molécule de protéine comprend la séquence représentée dans SEQ ID N° 1 ou un variant fonctionnel de celle-ci,
(b) un produit de transcription d'une molécule d'ADN codant pour une molécule de protéine dont la fonction est de protéger des cellules contre la dégénérescence et/ou la mort cellulaire, dans lequel la séquence d'acides aminés de la molécule de protéine comprend la séquence représentée dans SEQ ID N° 1 ou un variant fonctionnel de celle-ci,
(c) une molécule de protéine dont la fonction est de protéger des cellules contre la dégénérescence et/ou la mort cellulaire, dans laquelle la séquence d'acides aminés de la molécule de protéine comprend la séquence représentée dans SEQ ID N° 1 ou un variant fonctionnel de celle-ci,
(d) une molécule d'ADN codant pour une molécule de protéine dont la fonction est de protéger des cellules contre la dégénérescence et/ou la mort cellulaire, capable de s'hybrider dans des conditions stringentes avec le complément de l'ADNc décrit dans SEQ ID N° 2,
(e) un produit de transcription d'une molécule d'ADN, codant pour une molécule de protéine dont la fonction est de protéger des cellules contre la dégénérescence et/ou la mort cellulaire, capable de s'hybrider dans des conditions stringentes avec le complément de l'ADNc décrit dans SEQ ID N° 2,
(f) un produit de traduction d'une molécule d'ADN, codant pour une molécule de protéine dont la fonction est de protéger des cellules contre la dégénérescence et/ou la mort cellulaire, capable de s'hybrider dans des conditions stringentes avec le complément de l'ADNc décrit dans SEQ ID N° 2,
et la comparaison dudit taux ou de ladite activité, ou à la fois dudit taux et de ladite activité, d'au moins l'une desdites substances (a) à (f) à une valeur de référence représentant un état de maladie ou de santé connu, pour effectuer l'évaluation d'un traitement d'une maladie chez ledit sujet.

24. La méthode selon l'une des revendications 21 à 23, dans laquelle une diminution d'un taux ou d'une activité de (i) un produit de transcription d'une molécule d'ADN codant pour une molécule de protéine dont la séquence d'acides aminés comprend la séquence représentée dans SEQ ID N° 1 ou un variant fonctionnel de celle-ci ou (ii) une molécule de protéine dont la séquence d'acides aminés comprend la séquence représentée dans SEQ ID N° 1 ou un variant fonctionnel de celle-ci, dans un échantillon prélevé audit sujet par rapport à une valeur de référence représentant un état de santé connu, indique la présence d'une maladie chez ledit sujet.

25. Les méthodes selon les revendications 21 à 24, dans lesquelles ladite maladie est une maladie neurologique.

26. La méthode selon l'une des revendications 21 à 25, dans laquelle ledit sujet souffre de la maladie d'Alzheimer ou d'affections neurofibrillaires associées, ou d'états neurodégénératifs **caractérisés par** la dégénérescence cellulaire ou la mort cellulaire, ou de la maladie de Parkinson, ou de la chorée de Huntington, ou de sclérose latérale amyotrophique ou de la maladie de Pick.

27. Une méthode pour identifier un agent qui affecte une activité, ou un taux, ou à la fois ladite activité et ledit taux, d'au moins une substance qui est choisie dans le groupe formé par
(a) une molécule d'ADN codant pour une molécule de protéine, dans laquelle la séquence d'acides aminés de la molécule de protéine comprend la séquence représentée dans SEQ ID N° 1 ou un variant fonctionnel de celle-ci,
(b) un produit de transcription d'une molécule d'ADN codant pour une molécule de protéine, dans lequel la séquence d'acides aminés de la molécule de protéine comprend la séquence représentée dans SEQ ID N° 1 ou un variant fonctionnel de celle-ci,
(c) une molécule de protéine dans laquelle la séquence d'acides aminés de la molécule de protéine comprend la séquence représentée dans SEQ ID N° 1 ou un variant fonctionnel de celle-ci,
(d) une molécule d'ADN capable de s'hybrider dans des conditions stringentes avec le complément de l'ADNe décrit dans SEQ ID N° 2,
(e) un produit de transcription d'une molécule d'ADN capable de s'hybrider dans des conditions stringentes avec le complément de l'ADNc décrit dans SEQ ID N° 2,
(f) un produit de traduction d'une molécule d'ADN capable de s'hybrider dans des conditions stringentes avec le complément de l'ADNe décrit dans SEQ ID N° 2,
comprenant les étapes de :
(i) fourniture d'un échantillon contenant au moins une substance qui est choisie dans le groupe formé par (a) à (f),
(ii) mise en contact dudit échantillon avec au moins un agent,
(iii) comparaison d'une activité, d'un taux, ou à la fois de ladite activité et dudit taux, d'au moins l'une desdites substances avant et après la mise en contact.

28. Une méthode de la revendication 27, dans laquelle la fonction de ladite molécule de protéine ou d'un variant de celle-ci est de protéger des cellules contre la dégénérescence et/ou la mort cellulaire.

29. Une méthode de la revendication 27 ou 28, dans laquelle ladite molécule d'ADN capable de s'hybrider avec le complément de l'ADNc décrit dans SEQ ID N° 2 code pour une molécule de protéine dont la fonction est de protéger des cellules contre la dégénérescence et/ou la mort cellulaire.

30. Une trousse pour le diagnostic ou le pronostic d'une maladie, ladite trousse comprenant :
(1) au moins un réactif qui est choisi dans le groupe formé par des réactifs qui détectent sélectivement
(a) une molécule d'ADN codant pour une molécule de protéine, dans laquelle la séquence d'acides aminés de la molécule de protéine comprend la séquence représentée dans SEQ ID N° 1 ou un variant fonctionnel de celle-ci,
(b) un produit de transcription d'une molécule d'ADN codant pour une molécule de protéine, dans lequel la séquence d'acides aminés de la molécule de protéine comprend la séquence représentée dans SEQ ID N° 1 ou un variant fonctionnel de celle-ci,
(c) une molécule de protéine, dans laquelle la séquence d'acides aminés de la molécule de protéine comprend la séquence représentée dans SEQ ID N° 1 ou un variant fonctionnel de celle-ci,
(d) une molécule d'ADN capable de s'hybrider dans des conditions stringentes avec le complément de l'ADNc décrit dans SEQ ID N° 2,
(e) un produit de transcription d'une molécule d'ADN, dans lequel ladite molécule d'ADN est capable de s'hybrider dans des conditions stringentes avec le complément de l'ADNc décrit dans SEQ ID N° 2,
(f) un produit de traduction d'une molécule d'ADN, dans lequel ladite molécule d'ADN est capable de s'hybrider dans des conditions stringentes avec le complément de l'ADNc décrit dans SEQ ID N° 2,
(2) des instructions pour établir le diagnostic ou le pronostic de ladite maladie par
(i) détection d'un taux, ou d'une activité, ou à la fois dudit taux et de ladite activité, d'au moins une substance qui est choisie dans le groupe formé par (a) à (f) dans un échantillon prélevé audit sujet ; et
(ii) établissement du diagnostic ou du pronostic de ladite maladie, dans lequel un taux ou une activité, ou à la fois ledit taux et ladite activité, d'au moins une substance qui est choisie dans le groupe formé par (a) à (f) qui sont modifiés comparativement à une valeur de référence représentant un état de santé connu ; ou un taux, ou une activité, ou à la fois ledit taux et ladite activité, d'au moins une substance qui est choisie dans le groupe formé par (a) à (f) qui sont similaires ou égaux à une valeur de référence représentant un état de maladie connu, indique le diagnostic ou le pronostic de ladite maladie.
